# EUROPEAN PATENT APPLICATION

(11) **EP 4 803 061 A2**
(43) Date of publication of application: **09.09.2026**
(21) Application number: 26155951.2
(22) Date of filing: 18.12.2023
(51) Int. Cl.: A61J 1/00

(54) **RECONSTITUTION AND INJECTION SYSTEM FOR SINGLE DOSE CONTRAST MEDIA**

(30) Priority: 19.12.2022 US 202263433741 P
(62) Document number(s) of the earlier application(s) in accordance with Art. 76 EPC: 23908258.9 / 4 637 670
(71) Applicant: Bayer HealthCare LLC, Whippany, NJ 07981 (US)
(72) Inventor: Kemper, Corey, Pittsburgh, Pennsylvania, 15206 (US); Volkar, John, Valencia, Pennsylvania, 16059 (US); Hu, Ellia, Eastwood, NSW, 2122 (AU); Croston, Tom, Ashfield, NSW, 2131 (AU); Ranzoni, Andrea, Crows Nest, NSW, 2065 (AU)
(74) Representative: BIP Patents

(57) **Abstract**

A fluid injector system for reconstituting a solute contrast media and injecting reconstituted contrast media solution resulting therefrom includes a first syringe containing a diluent, a container containing the solute contrast media, a first valve configured to provide selective fluid communication between the container and the first syringe, and a controller. The controller is programmed or configured to deliver the diluent into the container to reconstitute the solute contrast media into a reconstituted contrast media solution, and deliver the reconstituted contrast media solution into an administration line configured to be fluidly connectable to a patient.

## Description

### CROSS-REFERENCE TO RELATED APPLICATION

This application claims the benefit of U.S. Provisional Patent Application No. 63/433,741, which was filed December 19, 2022, the disclosure of which is incorporated by reference in its entirety.

### BACKGROUND

### Field of the Disclosure

The present disclosure relates to fluid injector systems, and more particularly to fluid injector systems, associated components, devices, and methods for reconstituting a solute contrast media and delivery of reconstituted contrast media solution resulting therefrom to a patient, which reduce cost and environmental impact by reducing weight, volume, and waste of shipped contrast media.

### Description of Related Art

Contrast media for computed tomography (CT) and other imaging procedures is a commodity type product for most imaging procedures. Conventionally, contrast media is supplied to care providers in liquid form (in some cases exceeding 50% water by weight) and stored as a liquid until needed for a procedure. The method of supply and delivery of contrast media has developed into two main camps. The first method, currently preferred by many care providers, focuses on using a single sterile disposable media (e.g. bottles, bags) for each patient. Any unused contrast media is then disposed of, and a subsequent patient is dosed with an entirely new disposable media. The second method focuses on using a larger volume sterile disposable media from which multiple patients can be dosed.

Current contrast media and injectors have inefficiencies which ultimately lead to waste and increased costs. There is significant waste generated in the disposable, single use approach that is currently desired by most care providers. Significant quantities of contrast media go unused prior to expiration and must be thrown away. As contrast media is relatively expensive, such waste is a non-trivial expense to care providers and, ultimately, their patients.

Further, contrast media is relatively heavy and consequently must be contained in relatively large storage vessels. This can complicate storage of the contrast media by a customer and require a customer to allocate a significant amount of space for storage of the contrast media. Shipping costs can also be significant due to the size and weight of the contrast media being transported to a customer. As noted above, manufactured contrast media may be over 50% water by weight, so much of these costs attributable to the presence of water which is not an active ingredient. Due to the nature of conventional liquid contrast media, such contrast media is most commonly transported in glass bottles, which are fragile and require protective packaging. The volume and mass of the water and glass are significant contributors to the cost associated with making, distributing, and using the contrast media.

Additionally, current methods of supplying contrast have significant disposal issues. Glass bottles with unused contrast media can be a significant source of waste and pose an environmental concern due to the iodine included in contrast media. Also, the contrast media has shelf life and stability issues, which in many cases drives the aforementioned use of bulky glass containers. Plastic containers generally cannot be used in place of glass because polymeric material (e.g. plastics) can adversely react with the contrast media, making the contrast media unsuitable for patient use. Moreover, liquid contrast media may crystallize regardless of the type of container in which it is stored (though suboptimal containers may expedite crystallization), which places a cap on the shelf life of the contrast media.

In view of the foregoing, there exists a need for fluid injector systems, associated components, devices, and methods for reconstituting a solute contrast media and delivery of the reconstituted contrast media resulting therefrom to a patient, which reduce cost and environmental impact by reducing weight, volume, and waste of shipped contrast media.

### SUMMARY

Accordingly, aspects of the present disclosure are directed to non-limiting embodiments of fluid injector systems and associated components for reconstituting a solute contrast media prior to injection, which reduce cost and environmental impact by reducing weight, volume, and waste of shipped contrast media.

According to an aspect of the disclosure, a fluid injector system for reconstituting a solute contrast media and injecting the reconstituted contrast media solution resulting therefrom includes a first syringe containing a diluent, a container containing the solute contrast media, a first valve configured to provide selective fluid communication between the container and the first syringe, and a controller. The controller is programmed or configured to deliver the diluent into the container to reconstitute the solute contrast media into the reconstituted contrast media solution, and deliver the reconstituted contrast media solution into an administration line configured to be fluidly connectable to a patient.

According to another aspect of the disclosure, a flow control assembly for a fluid injector system for reconstituting a solute contrast media and injecting a reconstituted contrast media solution resulting therefrom includes a first syringe, a second syringe, an administration line, and a container containing the solute contrast media. The container is comprised of a flexible membrane, a polymeric material capable of withstanding temperatures of at least 150 degrees Celsius, and an internal valve configured to receive a spike of a contrast line and to passively seal the container when the spike is removed from the internal valve. The assembly further includes a first valve configured to provide selective fluid communication between one or more of a diluent vessel containing a diluent, the container, the first syringe, and the administration line and a second valve configured to provide selective fluid communication between one or more of a flushing agent vessel containing a flushing agent, the second syringe, and the administration line. The assembly further includes an agitator configured to receive the container and comprised of a drive mechanism configured to agitate the container during reconstitution and at least one heating element configured to apply heat to the container, and a controller. The controller is programmed or configured to control the first valve to deliver the diluent from the diluent vessel into the container to reconstitute the solute contrast media into the reconstituted contrast media solution, deliver the reconstituted contrast media solution into the administration line configured to be fluidly connectable to a patient, and deliver the reconstituted contrast media solution into the patient. The controller is further programmed or configured to control the second valve to deliver the flushing agent from the flushing agent vessel into the administration line, and deliver the flushing agent into the patient. The container is further heat treated such that the solute contrast media reaches a temperature of at least 150 degrees Celsius prior to the agitator receiving the container.

According to another aspect of the disclosure a flow control assembly for a fluid injector system for reconstituting a solute contrast media and injecting a reconstituted contrast media solution resulting therefrom includes a first syringe, a second syringe, a third syringe, a filter comprising at least one filter media, an administration line, a container containing the solute contrast media. The assembly further includes a first valve configured to provide selective fluid communication between one or more of the container, a diluent vessel containing a diluent, the first syringe, and the filter; a second valve configured to provide selective fluid communication between one or more of the filter, the second syringe, and the administration line; and a third valve configured to provide selective fluid communication between one or more of a flushing agent vessel containing a flushing agent, the third syringe, and the administration line. The assembly further includes an agitator configured to receive the container and comprised of a drive mechanism configured to agitate the container during reconstitution, and a controller. The controller is programmed or configured to control the first valve to deliver the diluent from the diluent vessel into the container, control the agitator to agitate the container to reconstitute the solute contrast media with the diluent into the reconstituted contrast media solution, and control the first valve to deliver the reconstituted contrast media solution into the filter. The controller is further programmed or configured to control the second valve to deliver the filtered reconstituted contrast media solution into the second syringe and to deliver the filtered reconstituted contrast media solution from the second syringe into the administration line. The controller is further programmed or configured to control the third valve to deliver the flushing agent from the flushing agent vessel into the third syringe and to deliver the flushing agent from the third syringe into the administration line. The administration line is configured to be fluidly connectable to a patient, and the controller is further programmed or configured to deliver the filtered reconstituted contrast media solution and the flushing agent from the administration line into the patient.

According to another aspect of the disclosure, a method for reconstituting a solute contrast media and injecting a reconstituted contrast media solution resulting therefrom using a fluid injector system includes fluidly connecting a diluent vessel containing a diluent to a first syringe of the fluid injector system, fluidly connecting a container containing the solute contrast media to the first syringe of the fluid injector system, delivering the diluent into the container via the first syringe to reconstitute the solute contrast media, agitating the container to reconstitute the solute contrast media into the reconstituted contrast media solution, delivering the reconstituted contrast media solution into the first syringe; and delivering the reconstituted contrast media solution from the first syringe into an administration line of the fluid injector system.

According to another aspect of the disclosure, a method for reconstituting a solute contrast media and injecting a reconstituted contrast media solution resulting therefrom using a fluid injector system includes fluidly connecting a diluent vessel containing diluent to a first syringe of the fluid injector system, fluidly connecting a flushing agent vessel containing a flushing agent to a second syringe of the fluid injector system; fluidly connecting a container containing the solute contrast media to the first syringe of the fluid injector system, and fluidly connecting a liquid contrast media vessel containing liquid contrast media to a third syringe of the fluid injector system. The method further includes delivering the flushing agent from the one or more flushing agent vessels into the second syringe, delivering the flushing agent from the second syringe into an administration line of the fluid injector system, delivering the liquid contrast media from the liquid contrast media vessel into the third syringe, and delivering the liquid contrast media from the third syringe into the administration line of the fluid injector system.

According to another aspect of the disclosure, a container containing a solute contrast media for reconstitution in a fluid injector system, the fluid injector system for use in controlling operation of first and second syringes and corresponding first and second valves in a manifold therefor includes a flexible membrane defining a cavity therein for containing the solute contrast media, the flexible membrane comprising a polymeric material capable of withstanding temperatures of at least 150 degrees Celsius and of enabling the cavity to expand in size, and an internal valve in communication with the cavity. The internal valve is further configured to receive a spike associated with the fluid injector system such that: (I) upon insertion of the spike into the internal valve, the internal valve opens and thereby under control of the first valve enables alternately: (a) a diluent to flow from the first syringe through the first valve and the internal valve into the cavity and mix with the solute contrast media to enable formation of a reconstituted contrast media solution therein; and (b) the reconstituted contrast media solution to flow from the cavity through the internal valve and the first valve into the first syringe from which the fluid injector system is capable of injecting the reconstituted contrast media solution through the first valve through an administration line into a patient; and (II) upon removal of the spike from the internal valve, the internal valve closes.

Non-limiting illustrative examples of embodiments of the present disclosure will now be described in the following numbered clauses.

Clause 1: A fluid injector system for reconstituting a solute contrast media and injecting a reconstituted contrast media solution resulting therefrom, the fluid injector system comprising: a first syringe containing a diluent; a container containing the solute contrast media; a first valve configured to provide selective fluid communication between the container and the first syringe; and a controller programmed or configured to: deliver the diluent into the container to reconstitute the solute contrast media into the reconstituted contrast media solution; and deliver the reconstituted contrast media solution into an administration line configured to be fluidly connectable to a patient.

Clause 2: The fluid injector system of clause 1, wherein the fluid injector system further comprises: a second syringe; and a second valve configured to provide selective fluid communication between one or more of a flushing agent vessel containing a flushing agent, the second syringe, and the administration line.

Clause 3: The fluid injector system of clause 1 or 2, wherein the controller is further programmed or configured to: deliver the flushing agent into the administration line.

Clause 4: The fluid injector system of any of clauses 1-3, wherein the first valve is further configured to provide selective fluid communication between one or more of a diluent vessel containing a diluent, the container, the first syringe, and the administration line.

Clause 5: The fluid injector system of any of clauses 1-4, wherein the fluid injector system further comprises an agitator configured to receive the container, wherein the agitator comprises a drive mechanism configured to agitate the container during reconstitution.

Clause 6: The fluid injector system of any of clauses 1-5, wherein the agitator further comprises at least one heating element configured to apply heat to the container, the solute contrast media, and the diluent contained within the container during reconstitution.

Clause 7. The fluid injector system of any of clauses 1-6, wherein the container is heat treated such that the solute contrast media reaches a temperature of at least 150 degrees Celsius and such that the solute contrast media is sterilized prior to the agitator receiving the container.

Clause 8: The injector system of any of clauses 1-7, wherein the container is heat treated such that the solute contrast media reaches a temperature of 200 degrees Celsius and such that the solute contrast media is both sterilized and cleaned via depyrogenation prior to the agitator receiving the container.

Clause 9. The fluid injector system of any of clauses 1-8, wherein the fluid injector system further comprises a filter between the container and the first valve, and wherein the filter comprises at least one filter media configured to filter the reconstituted contrast media solution.

Clause 10: The fluid injector system of any of clauses 1-9, wherein the filter is positively charged to remove endotoxins from the reconstituted contrast media solution.

Clause 11: The fluid injector system of any of clauses 1-10, wherein the fluid injector system further comprises a pump configured to be in fluid communication with the container, the filter and the first syringe, and wherein the pump and the filter are positioned between a spike of a contrast line and the first valve.

Clause 12: The fluid injector system of any of clauses 1-11, wherein the fluid injector system further comprises: a second syringe; a third syringe; a filter comprising at least one filter media configured to filter the reconstituted contrast media solution, wherein the first valve is further configured to provide selective fluid communication between one or more of the container, the first syringe, a diluent vessel containing the diluent, and the filter, a second valve configured to provide selective fluid communication between one or more of the filter, the second syringe, and the administration line; and a third valve configured to provide selective fluid communication between one or more of a flushing agent vessel containing a flushing agent, the third syringe, and the administration line, wherein the controller is further programmed or configured to: deliver the reconstituted contrast media solution from the container to the first syringe, from the first syringe to the filter, and from the filter to the second syringe; deliver the filtered reconstituted contrast media solution from the second syringe to the administration line; deliver the flushing agent from the flushing agent vessel into the third syringe, and deliver the flushing agent from the third syringe to the administration line.

Clause 13: The fluid injector system of any of clauses 1-12, wherein the container comprises: a flexible membrane comprising a polymeric material capable of withstanding temperatures of at least 150 degrees Celsius and configured to expand; and an internal valve configured to receive a spike of a contrast line and configured to be in fluid communication with the first valve.

Clause 14: The fluid injector system of any of clauses 1-13, wherein the internal valve is configured to passively seal the container when the spike is removed from the internal valve.

Clause 15: The fluid injector system of any of clauses 1-14, wherein the controller is further programmed or configured to: deliver an unused portion of the reconstituted contrast media solution from the first syringe into the container.

Clause 16: The fluid injector system of any of clauses 1-15, further comprising: a third syringe; and a third valve configured to provide fluid communication between one or more of the third syringe, a liquid contrast media vessel, and the administration line.

Clause 17: The fluid injector system of any of clauses 1-16, wherein the controller is further programmed or configured to: deliver a liquid contrast media contained in the liquid contrast media vessel into the administration line.

Clause 18: The fluid injector system of any of clauses 1-17, wherein the controller is further programmed or configured to: deliver an unused portion of the reconstituted contrast media solution from the first syringe into the third syringe; and deliver the unused portion of the reconstituted contrast media solution from the third syringe into the liquid contrast media vessel.

Clause 19: The fluid injector system of any of clauses 1-18, wherein the fluid injector system further comprises at least one reader, wherein the container comprises at least one readable solute contrast media identifier, the flushing agent vessel comprises at least one readable flushing agent identifier, and the diluent vessel comprises at least one readable diluent identifier, wherein the at least one reader is configured to scan and read the at least one readable solute contrast media identifier, the at least one readable flushing agent identifier, and the at least one readable diluent identifier and communicate identifier information with the controller, wherein the controller is further programmed or configured to process the identifier information and to start and stop fluid communication between one or more of the diluent vessel, the container, the first syringe, and the administration line, and between one or more of the flushing agent vessel, the second syringe, and the administration line based on the identifier information.

Clause 20: A flow control assembly for a fluid injector system for reconstituting a solute contrast media and injecting a reconstituted contrast media solution resulting therefrom, the flow control assembly comprising: a first syringe; a second syringe; an administration line; a container containing the solute contrast media and comprised of a flexible membrane, a polymeric material capable of withstanding temperatures of at least 150 degrees Celsius, and an internal valve configured to receive a spike of a contrast line and to passively seal the container when the spike is removed from the internal valve; a first valve configured to provide selective fluid communication between one or more of a diluent vessel containing a diluent, the container, the first syringe, and the administration line; a second valve configured to provide selective fluid communication between one or more of a flushing agent vessel containing a flushing agent, the second syringe, and the administration line; an agitator configured to receive the container and comprised of a drive mechanism configured to agitate the container during reconstitution and at least one heating element configured to apply heat to the container; and a controller programmed or configured to: control the first valve to deliver the diluent from the diluent vessel into the container to reconstitute the solute contrast media into the reconstituted contrast media solution, deliver the reconstituted contrast media solution into the administration line configured to be fluidly connectable to a patient, and deliver the reconstituted contrast media solution into the patient; and control the second valve to deliver the flushing agent from the flushing agent vessel into the administration line, and deliver the flushing agent into the patient, wherein the container is heat treated such that the solute contrast media reaches a temperature of at least 150 degrees Celsius prior to the agitator receiving the container.

Clause 21: A flow control assembly for a fluid injector system for reconstituting a solute contrast media and injecting a reconstituted contrast media solution resulting therefrom, the flow control assembly comprising: a first syringe; a second syringe; a third syringe; a filter comprising at least one filter media; an administration line; a container containing the solute contrast media; a first valve configured to provide selective fluid communication between one or more of the container, a diluent vessel containing a diluent, the first syringe, and the filter; a second valve configured to provide selective fluid communication between one or more of the filter, the second syringe, and the administration line, a third valve configured to provide selective fluid communication between one or more of a flushing agent vessel containing a flushing agent, the third syringe, and the administration line; an agitator configured to receive the container and comprised of a drive mechanism configured to agitate the container during reconstitution; and a controller programmed or configured to: control the first valve to deliver the diluent from the diluent vessel into the container, control the agitator to agitate the container to reconstitute the solute contrast media with the diluent into the reconstituted contrast media solution, and control the first valve to deliver the reconstituted contrast media solution into the filter; control the second valve to deliver the filtered reconstituted contrast media solution into the second syringe and to deliver the filtered reconstituted contrast media solution from the second syringe into the administration line; and control the third valve to deliver the flushing agent from the flushing agent vessel into the third syringe and to deliver the flushing agent from the third syringe into the administration line, wherein the administration line is configured to be fluidly connectable to a patient, and wherein the controller is further programmed or configured to deliver the filtered reconstituted contrast media solution and the flushing agent from the administration line into the patient.

Clause 22: A method for reconstituting a solute contrast media and injecting a reconstituted contrast media solution resulting therefrom using a fluid injector system, the method comprising: fluidly connecting a diluent vessel containing a diluent to a first syringe of the fluid injector system; fluidly connecting a container containing the solute contrast media to the first syringe of the fluid injector system; delivering the diluent into the container via the first syringe to reconstitute the solute contrast media; agitating the container to reconstitute the solute contrast media into the reconstituted contrast media solution; delivering the reconstituted contrast media solution into the first syringe; and delivering the reconstituted contrast media solution from the first syringe into an administration line of the fluid injector system.

Clause 23: The method of clause 22, wherein the method further comprises sterilizing the solute contrast media by heating the solute contrast media in the container to a temperature of at least 150 degrees Celsius before fluidly connecting the container to the first syringe.

Clause 24: The method of clause 22 or 23, wherein the method further comprises cleaning the reconstituted contrast media solution by passing the reconstituted contrast media solution through a filter comprising at least one filter media before delivering the reconstituted contrast media solution into the administration line of the fluid injector system.

Clause 25. The method of any of clauses 22-24, wherein the method further comprises: fluidly connecting a flushing agent vessel containing a flushing agent to a second syringe of the fluid injector system; and delivering the flushing agent from the flushing agent vessel into the second syringe; and delivering the flushing agent from the second syringe into the administration line of the fluid injector system.

Clause 26: The method of any of clauses 22-25, wherein the method further comprises: purging the fluid injector system of air prior to fluidly connecting the container containing the solute contrast media to the first syringe of the fluid injector system.

Clause 27: The method of any of clauses 22-26, wherein the method further comprises: fluidly connecting a flushing agent vessel containing a flushing agent to a second syringe of the fluid injector system; and priming the administration line with at least one of the diluent from the diluent vessel and the flushing agent from the flushing agent vessel prior to connecting the container containing the solute contrast media to the first syringe of the fluid injector system.

Clause 28: The method of any of clauses 22-27, further comprising: delivering an unused portion of the reconstituted contrast media solution from the first syringe into the container.

Clause 29: The method of any of clauses 22-28, further comprising: disconnecting the container containing the unused portion of the reconstituted contrast media solution from the fluid injector system; and sending the container to a recycling facility to reclaim an active ingredient of the reconstituted contrast media solution.

Clause 30: The method of any of clauses 22-29 further comprising: delivering an unused portion of the reconstituted contrast media solution from the first syringe into a liquid contrast media vessel.

Clause 31: The method of any of clauses 22-30, further comprising: determining, with a controller of the fluid injector system, whether the container is approved for use in an injection procedure based on at least one factor.

Clause 32: The method of any of clauses 22-31, wherein the at least one factor comprises at least one of: an expiration date of the container; the solute contrast media not being suitable for the injection procedure; the container containing an unapproved substance; the container not containing an amount of the solute contrast media sufficient to compete the injection procedure; and the container having previously been used.

Clause 33: The method of any of clauses 22-32, further comprising: aborting the injection procedure in response to the controller determining that the container is not approved for use in the injection procedure.

Clause 34. A method for reconstituting a solute contrast media and injecting a reconstituted contrast media solution resulting therefrom using a fluid injector system, the method comprising: fluidly connecting a diluent vessel containing diluent to a first syringe of the fluid injector system; fluidly connecting a flushing agent vessel containing a flushing agent to a second syringe of the fluid injector system; fluidly connecting a container containing the solute contrast media to the first syringe of the fluid injector system; fluidly connecting a liquid contrast media vessel containing liquid contrast media to a third syringe of the fluid injector system; delivering the flushing agent from the one or more flushing agent vessels into the second syringe; delivering the flushing agent from the second syringe into an administration line of the fluid injector system; delivering the liquid contrast media from the liquid contrast media vessel into the third syringe; and delivering the liquid contrast media from the third syringe into the administration line of the fluid injector system.

Clause 35: A container containing a solute contrast media for reconstitution in a fluid injector system, the fluid injector system for use in controlling operation of first and second syringes and corresponding first and second valves in a manifold therefor, the container comprising: a flexible membrane defining a cavity therein for containing the solute contrast media, the flexible membrane comprising a polymeric material capable of withstanding temperatures of at least 150 degrees Celsius and of enabling the cavity to expand in size; and an internal valve in communication with the cavity and configured to receive a spike associated with the fluid injector system such that: (I) upon insertion of the spike into the internal valve, the internal valve opens and thereby under control of the first valve enables alternately: (a) a diluent to flow from the first syringe through the first valve and the internal valve into the cavity and mix with the solute contrast media to enable formation of a reconstituted contrast media solution therein; and (b) the reconstituted contrast media solution to flow from the cavity through the internal valve and the first valve into the first syringe from which the fluid injector system is capable of injecting the reconstituted contrast media solution through the first valve through an administration line into a patient; and (II) upon removal of the spike from the internal valve, the internal valve closes. Further details and advantages of the various examples described in detail herein will become clear upon reviewing the following detailed description of the various examples in conjunction with the accompanying drawing figures.

### BRIEF DESCRIPTION OF THE DRAWINGS

Additional advantages and details are explained in greater detail below with reference to the exemplary embodiments that are illustrated in the accompanying schematic figures, in which:
FIG. 1 is a schematic of a non-limiting embodiment of a fluid injector system in accordance with an aspect or example of the present disclosure;
FIG. 2 is a schematic of the fluid injector system of FIG. 1 in a first position;
FIG. 3 is a schematic of the fluid injector system of FIG. 1 in a second position;
FIG. 4 is a schematic of the fluid injector system of FIG. 1 in a third position;
FIG. 5 is a schematic of the fluid injector system of FIG. 1 in a fourth position;
FIG. 6 is a schematic of the fluid injector system of FIG. 1 in a fifth position;
FIG. 7 is a schematic of the fluid injector system of FIG. 1 in a sixth position;
FIG. 8 is a schematic of the fluid injector system of FIG. 1 in a seventh position;
FIG. 9 is a schematic of the fluid injector system of FIG. 1 in an eighth position;
FIG. 10A is a side perspective view of a non-limiting embodiment of syringes, valves and a distribution manifold of a fluid injector system in accordance with an aspect or example of the present disclosure;
FIG. 10B is a side view of a non-limiting embodiment of syringes, valves, a distribution manifold, tubes, a filter, and a spike of a fluid injector system in accordance with an aspect or example of the present disclosure;
FIG. 11 is a front sectional view of a non-limiting embodiment of a fluid injector system in accordance with an aspect or example of the present disclosure;
FIG. 12 is a side perspective view of a non-liming embodiment of flow control assembly of a fluid injector system in accordance with an aspect or example of the present disclosure;
FIG. 13A is a front perspective view of a non-limiting embodiment of a fluid injector system in accordance with an aspect or example of the present disclosure;
FIG. 13B is a side sectional view of a non-limiting embodiment of a fluid injector system in accordance with an aspect or example of the present disclosure;
FIG. 14 is a top sectional view of a non-limiting embodiment of an agitator of a fluid injector system in accordance with an aspect or example of the present disclosure;
FIG. 15 is a schematic of a non-limiting embodiment of a fluid injector system in accordance with an aspect or example of the present disclosure;
FIG. 16A is a perspective view of a non-limiting embodiment of a filter of a fluid injector system in accordance with an aspect or example of the present disclosure;
FIG. 16B is a perspective view of a non-limiting embodiment of a filter of a fluid injector system in accordance with an aspect or example of the present disclosure;
FIG. 17 is a schematic of a non-limiting embodiment of a fluid injector system in accordance with an aspect or example of the present disclosure;
FIG. 18 is a schematic of the fluid injector system of FIG. 17 in a first position;
FIG. 19 is a schematic of the fluid injector system of FIG. 17 in a second position;
FIG. 20 is a schematic of the fluid injector system of FIG. 17 in a third position;
FIG. 21 is a schematic of the fluid injector system of FIG. 17 in a fourth position;
FIG. 22 is a schematic of the fluid injector system of FIG. 17 in a fifth position;
FIG. 23 is a schematic of the fluid injector system of FIG. 17 in a sixth position;
FIG. 24 is a schematic of the fluid injector system of FIG. 17 in a seventh position;
FIG. 25 is a schematic of the fluid injector system of FIG. 17 in an eighth position;
FIG. 26A is a perspective view of a non-limiting embodiment of a container for containing solute contrast media of a fluid injector system in accordance with an aspect or example of the present disclosure;
FIG. 26B is an exploded perspective view of the container of FIG. 26A;
FIG. 27A is a perspective view of a non-limiting embodiment of a container for containing solute contrast media of a fluid injector system in accordance with an aspect or example of the present disclosure;
FIG. 27B is a side view of the container of FIG. 27A;
FIG. 27C is a perspective view of Detail A of the container of FIG. 27A;
FIG. 28 is a side perspective view of the container of FIGS. 27A and 27B being connected to a contrast tube of a fluid injector system in accordance with an aspect or example of the present disclosure;
FIG. 29A is a top plan view of the container of FIGS. 27A-27C, with fluid being supplied thereto;
FIG. 29B is a top plan view of the container of FIGS. 27A-27C, with fluid being withdrawn therefrom;
FIG. 29C is a top plan view of the container of FIGS. 27A-27C being disconnected from the contrast tube;
FIG. 30A is a side view of a spike fully inserted in the container of FIGS. 27A-27C in accordance with an aspect or example of the present disclosure;
FIG. 30B is a side view of the spike partially inserted in the container of FIGS. 27A-27C;
FIG. 30C is a side view of the spike being removed from the container of FIGS. 27A-27C;
FIG. 31A is a top plan view of a non-limiting embodiment of a distribution manifold of a fluid injector system in accordance with an aspect for example of the present disclosure;
FIG. 31B is a perspective view of a non-limiting embodiment of a distribution manifold of a fluid injector system in accordance with an aspect or example of the present disclosure;
FIG. 32A is a side sectional view of a non-limiting embodiment of a valve of a fluid injector system in accordance with an aspect or example of the present disclosure;
FIG. 32B is a perspective view of a non-limiting embodiment of a valve of a fluid injector system in accordance with an aspect or example of the present disclosure;
FIG. 33A is a perspective view of a non-limiting embodiment of an agitator of a fluid injector system in accordance with an aspect or example of the present disclosure;
FIG. 33B is a side view of the agitator of FIG. 33A;
FIG. 33C is a perspective view of the agitator of FIG. 33A in a retracted position in accordance with an aspect or example of the present disclosure;
FIG. 33D is a perspective view of the agitator of FIG. 33A in an extended position in accordance with an aspect or example of the present disclosure;
FIG. 34 is a side view of a non-limiting embodiment of a housing, stand, and agitator of a fluid injector system in accordance with an aspect or example of the present disclosure;
FIG. 35A is a side perspective view of the agitator of FIG. 34;
FIG. 35B is a top perspective view of the agitator of FIG. 34;
FIG. 36 is a schematic of the agitator of FIG. 34;
FIG. 37A is side view of the agitator of FIG. 34;
FIG. 37B is a perspective view of a counterweight of the agitator of FIG. 34;
FIG. 38 is a schematic of a non-limiting embodiment of a fluid injector system in accordance with an aspect or example of the present disclosure;
FIG. 39 is a schematic of the fluid injector system of FIG. 38 in a first position;
FIG. 40 is a schematic of the fluid injector system of FIG. 38 in a second position;
FIG. 41 is a schematic of the fluid injector system of FIG. 38 in a third position;
FIG. 42 is a schematic of the fluid injector system of FIG. 38 in a fourth position;
FIG. 43 is a schematic of the fluid injector system of FIG. 38 in a fifth position;
FIG. 44 is a schematic of the fluid injector system of FIG. 38 in a sixth position;
FIG. 45 is a schematic of the fluid injector system of FIG. 38 in a seventh position;
FIG. 46 is a schematic of the fluid injector system of FIG. 38 in an eight position;
FIG. 47 is a schematic of the fluid injector system of FIG. 1 in a ninth position;
FIG. 48 is a schematic of the fluid injector system of FIG. 1 in a tenth position;
FIG. 49 is a sequence diagram of a non-limiting embodiment of a method for reconstituting a solute contrast media and injecting the reconstituted contrast media solution resulting therefrom using a fluid injector system in accordance with an aspect or example of the present disclosure; and
FIG. 50 is a sequence diagram of a non-limiting embodiment of a method for validating components of a fluid injector system in accordance with an aspect or example of the present disclosure.

Corresponding reference characters indicate corresponding parts throughout the several views. The exemplifications set out herein illustrate exemplary embodiments of the disclosure, and such embodiments are not to be construed as limiting the scope of the disclosure in any manner.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects of the disclosure. Hence, specific dimensions and other physical characteristics related to the embodiments or aspects disclosed herein are not to be considered as limiting unless otherwise indicated.

For purposes of the description hereinafter, the terms "end," "upper," "lower," "right," "left," "vertical," "horizontal," "top," "bottom," "lateral," "longitudinal," and derivatives thereof shall relate to embodiments or aspects of the disclosure as they are oriented in the drawing figures. However, it is to be understood that embodiments or aspects may assume various alternative variations and step sequences, except where expressly specified to the contrary. Spatial or directional terms, such as "left", "right", "inner", "outer", "above", "below", and the like, are not to be considered as limiting as the disclosure can assume various alternative orientations.

All numbers used in the specification and claims are to be understood as being modified in all instances by the term "about". The terms "approximately", "about", and "substantially" mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." As used in the specification and the claims, the singular form of "a," "an," and "the" include plural referents, unless the context clearly dictates otherwise. Where only one item is intended, the term "one" or similar language is used. Also, as used herein, the terms "has," "have," "having," or the like are intended to be openended terms. Further, the phrase "based on" is intended to mean "based at least partially on" unless explicitly stated otherwise. Further, the phrase "based on" is intended to mean "based at least partially on" unless explicitly stated otherwise.

When used in relation to a component of a fluid injector system such as a fluid reservoir, a syringe, or a fluid line, the term "distal" refers to a portion of said component nearest to a patient. When used in relation to a component of a fluid injector system such as a fluid reservoir, a syringe, or a fluid line, the term "proximal" refers to a portion of said component nearest to the injector of the fluid injector system (i.e. the portion of said component farthest from the patient). When used in relation to a syringe of a multi-patient disposable set, the term "proximal" refers to a portion of a syringe nearest a piston for delivering fluid from a syringe. When used in relation to a component of a fluid injector system such as a fluid reservoir, a syringe, or a fluid line, the term "upstream" refers to a direction away from the patient and towards the injector of the fluid injector system. For example, if a first component is referred to as being "upstream" of a second component, the first component is located nearer to the injector than the second component is to the injector. When used in relation to a component of a fluid injector system such as a fluid reservoir, a syringe, or a fluid line, the term "downstream" refers to a direction towards the patient and away from the injector of the fluid injector system. For example, if a first component is referred to as being "downstream" of a second component, the first component is located nearer to the patient than the second component is to the patient.

Embodiments of the present disclosure are generally directed to a fluid injector system for reconstituting a solute contrast media and injecting the reconstituted contrast media solution resulting therefrom, the fluid injector system may include a first syringe containing a diluent, a container containing the solute contrast media, a first valve configured to provide selective fluid communication between the container and the first syringe, and a controller. The controller may be programmed or configured to deliver the diluent into the container to reconstitute the solute contrast media into the reconstituted contrast media solution, and deliver the reconstituted contrast media solution into an administration line configured to be fluidly connectable to a patient.

In this way, embodiments of the present disclosure allow for fluid injector systems, associated components, devices, and methods for reconstituting a solute contrast media and delivery of reconstituted contrast media solution resulting therefrom to a patient, which reduce cost and environmental impact by reducing weight, volume, and waste of shipped contrast media.

Referring to the drawings in which like reference characters refer to like parts throughout the several views thereof, the present disclosure is generally directed to systems, associated components, devices, and methods for reconstituting a solute contrast media and delivery of reconstituted contrast media solution resulting therefrom. While the present disclosure is generally described in connection with computed tomography (CT) imaging procedures, the systems, devices, and methods described herein may also be used in applications outside of CT where reconstitution and intravenous injection of contrast media is indicated. Such procedures include but are not limited to X-ray, magnetic resonance imaging (MRI), and ultrasound procedures. It is also to be understood that the systems, devices, and methods disclosed herein are also applicable to reconstitution and injection of other solutes such as therapeutic agents, medicaments, drugs, and the like. It is to be further understood that that the solute may be in various physical states or forms. For example, the solute may be a solid, such as a dry powder, or it may be a concentrated liquid.

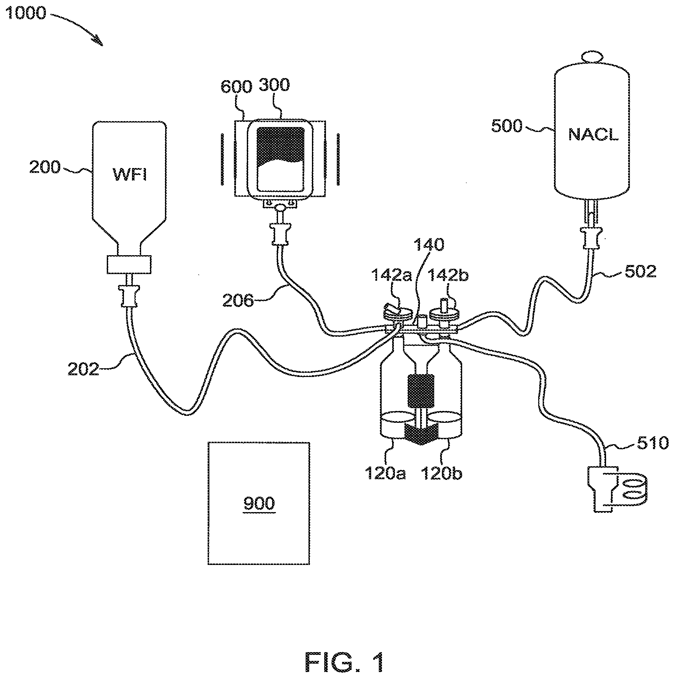

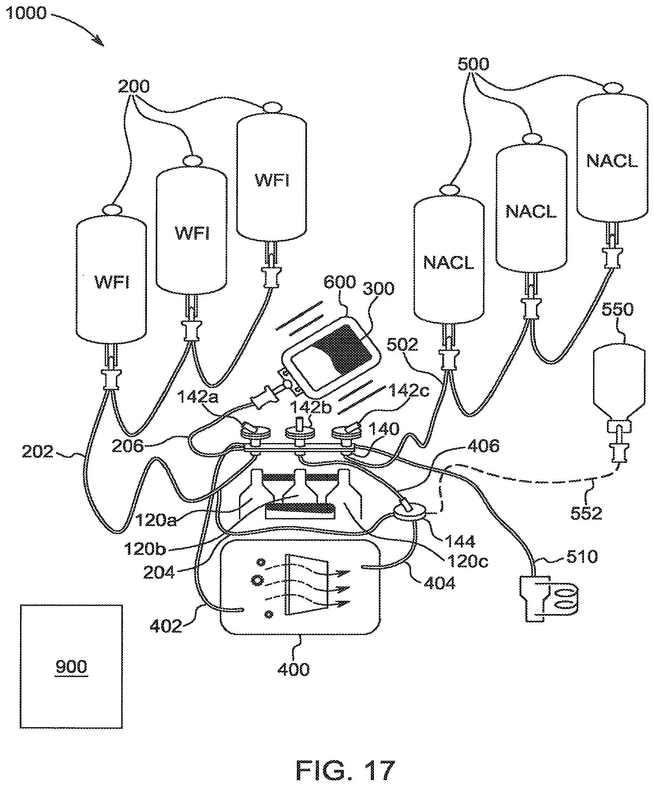

Referring first to FIG. 1, FIG. 1 shows a schematic of a non-limiting embodiment of fluid injector system 1000 for reconstituting and injecting a solute contrast media in accordance with the present disclosure. Fluid injector system 1000 may include a flow control assembly having one or more syringes 120a, 120b fluidly connected to a distribution manifold 140. The distribution manifold 140 may include a plurality of input/output ports that may be opened and closed by corresponding valves 142a, 142b, thereby providing selective fluid communication between the various components of the system 1000 as described herein. While the exemplary fluid injector system 1000 illustrated in FIG. 1 includes two syringes 120a, 120b and two valves, 142a, 142b, the system 1000 may include more or less of these components as necessary to perform a desired injection procedure. For example, in addition to the two barrel configuration depicted in FIG. 1, the present disclosure also contemplates a three barrel configuration and associated valve and manifold architectures and componentry as shown and discussed with reference to FIGS 17-25 and 38-46.

Accordingly, fluid injector system 1000 may include first syringe 120a containing a diluent (e.g. water-for-injection), container 300 containing solute contrast media, and first valve 142a configured to provide selective fluid communication between container 300 and first syringe 120a. System 1000 may further include controller 900 programmed or configured to the deliver the diluent into container 300 to reconstitute the solute contrast media into a reconstituted contrast media solution, and deliver the reconstituted contrast media solution into administration line 510 configured to be fluidly connectable to a patient.

In the exemplary fluid injection system shown in FIG. 1, syringes 120a, 120b, distribution manifold 140, and valves 142a, 142b may be configured as a "day set" or "multiuse disposable set (MUDS)" - an assembly that is changed out once per day and may be used to perform multiple injection procedures on multiple patients. Examples of syringes 120a, 120b and associated housings and electromechanical components for operating syringes 120a, 120b are described in U.S. Patent No. 10,507,319, the disclosure of which is hereby incorporated by reference in its entirety. It is noted, however, that features of distribution manifold 140 of the present disclosure, as described in connection with FIGS. 1-9, 15, 17-25, 31-32, and 30-48 herein, are not disclosed in U.S. Patent No. 10,507,319.

System 1000 may further include a diluent vessel such as water-for-injection (WFI) vessel 200 shown in FIG. 1. Distribution manifold 140 may be fluidly connected to one or more water-for-injection (WFI) vessels 200 containing a diluent such as water or another fluid suitable for reconstituting dry (e.g. powdered) solute contrast media. First valve 142a may be configured to provide selective fluid communication between one or more WFI vessel 200, container 300, first syringe 142a, and administration line 510.

Ideally, the water for use in WFI vessel 200 should be sterile, non-pyrogenic, distilled water in a single dose container for intravenous administration after addition of a suitable solute. WFI vessel 200 may also be used as a dispensing container for diluent use. Preferably, no antimicrobial or other substance should be added to the WFI. Furthermore, the WFI should exhibit a pH range from 5.0 to 7.0 though preferably 5.5, and an osmolarity of 0. WFI comes in commonly available in bulk volumes, i.e., 0.5 L or 1.0 L bags. In particular, the one or more WFI vessels 200 are fluidly connected to first valve 142a of distribution manifold 140 via main water tube 202.

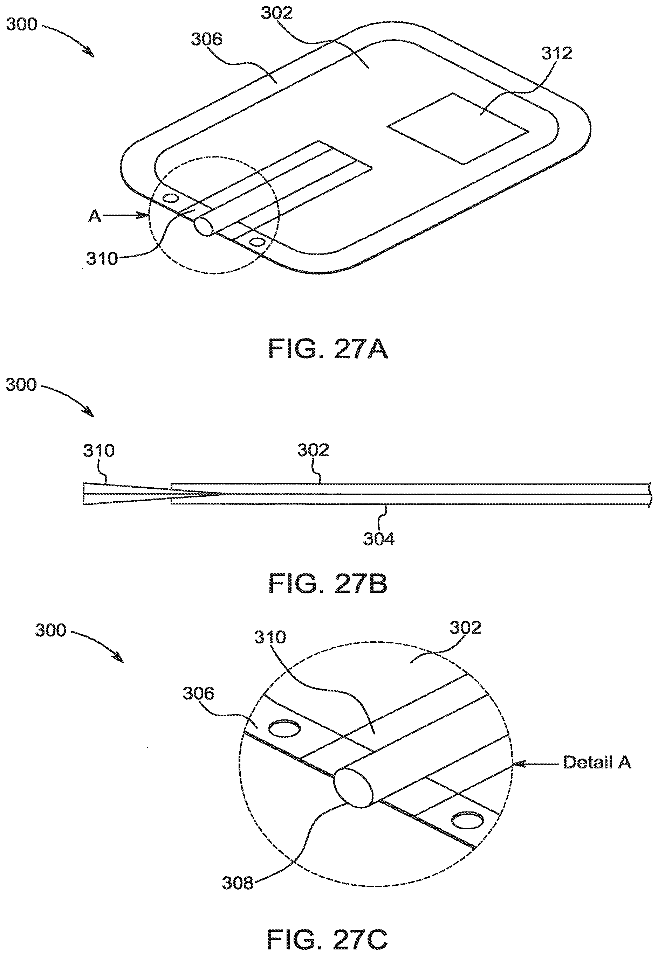

Container 300 containing the solute contrast media may be pouch 300, described below with reference to FIGS. 27A-28, or another suitable container. Pouch 300 may contain a therapeutic agent (e.g. contrast media) in dry (e.g., powdered) form and may include valve 310 that is fluidly connected to first valve 142a of distribution manifold 140 via contrast tube 206. System 1000 may include agitator 600 configured to receive container 300. Agitator 600 may include a drive mechanism and/or other mechanical features to shake, agitate, rotate, translate, etc. pouch 300 during reconstitution of the contrast media therein. Non-mechanical means (e.g. heat) may also be used to deliver energy to the mix of powered media and WFI to assure adequate reconstitution of the contrast media solution. In some non-limiting embodiments, agitator 600 may include at least one heating element configured to apply heat to container 300 and the solute contrast media within during reconstitution. In some non-limiting embodiments, agitator 600 may be configured to facilitate other non-mechanical means of energy transfer to container 300 for reconstitution, instead of or in addition to mechanical and/or heat energy. For example, agitator 600 may be configured to deliver electrical, magnetic, electromagnetic, light, sound, radiation or another energy to container 300 for reconstitution.

System 100 may also include second syringe 120b and second valve 142b configured to provide selective fluid communication between one or more of flushing agent vessel 500 containing a flushing agent, second syringe 120b, and administration line 510. Accordingly, controller 900 may be further programmed or configured to deliver the flushing agent into administration line 510. Flushing agent vessel may contain, for example, saline containing sodium chloride (NaCl), for use in flushing/priming system 1000. Administration line 510 may be an infusion set for connection to a patient. The administration line/infusion set 510 may include, for example, a needle for insertion into a vein of a patient.

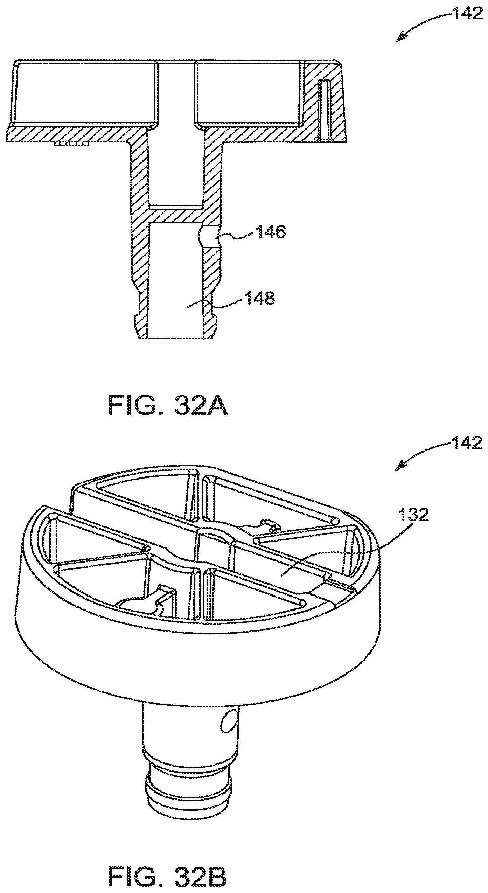

Each of valves 142a, 142b may be a multi-position stopcock, for example, as shown in FIGS. 32A and 32B, configured to facilitate selective control of fluid flow between the various system components connected thereto. First valve 142a may facilitate selective fluid flow control between WFI vessel(s) 200, pouch 300, first syringe 120a, and administration line 510. Second valve 142b may facilitate selective fluid flow control between second syringe 120b, flushing agent vessel 500, and administration line 510.

The controller 900 may be configured to control operation of various components of injector system 1000 described herein. Controller 900 includes at least one processor and is configured to perform one or more operations of fluid injector system 1000 according to one or more injection protocols stored in a memory of or accessible by controller 900.

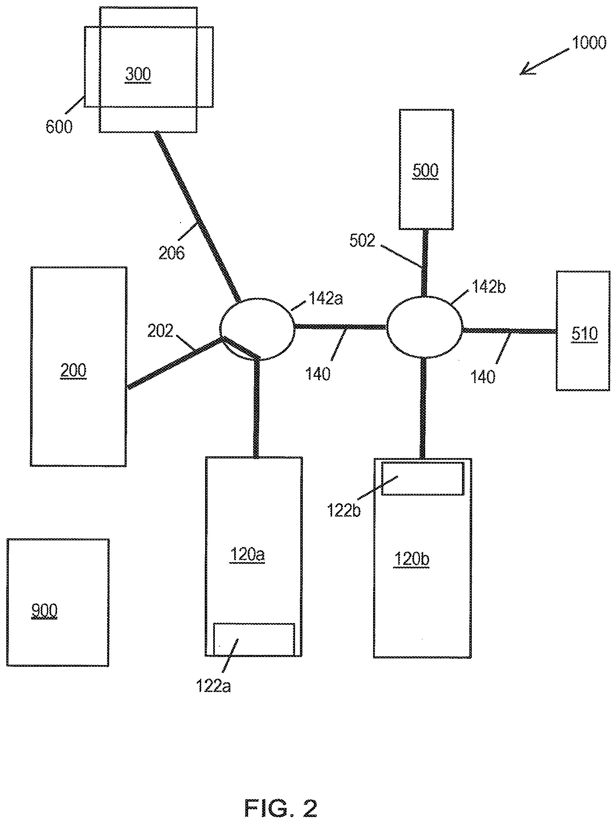

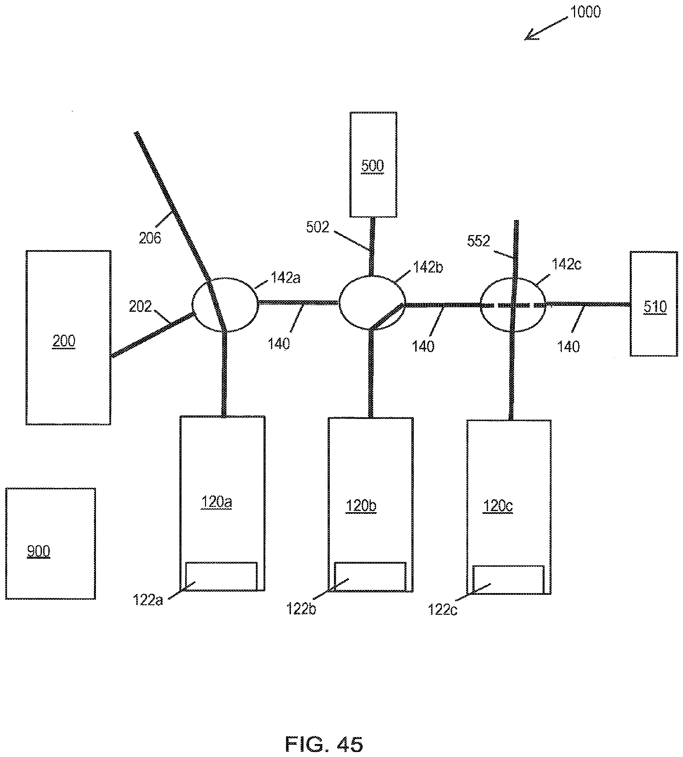

In order to ultimately deliver a predetermined dose of contrast media to the patient, the dry contrast media contained in the pouch 300 must first be reconstituted to obtain contrast media in liquid form as an injectable (i.e. safe for injection into patient) contrast media solution. Reconstitution and injection of the contrast media is achieved by operating syringes 120a, 120b and valves 142a, 142b in a prescribed sequence as shown in FIGS. 2-9. The following description of FIGS. 2-9 omits steps of purging/priming system 1000, as these operations are typically performed only once per day for the system, and therefore purging and priming is not representative of the majority of uses of the system. Details of the purging and priming operations are described in detail herein with reference to FIGS. 45-48.

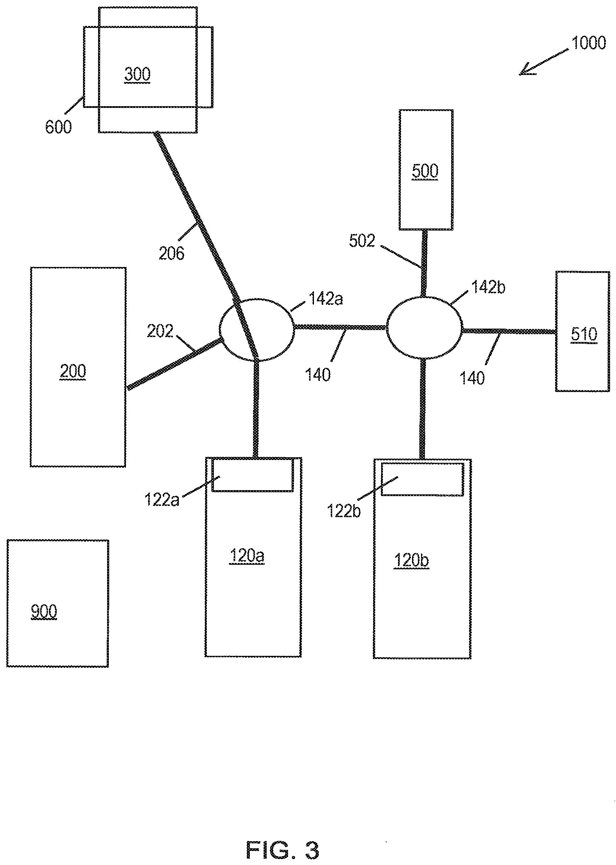

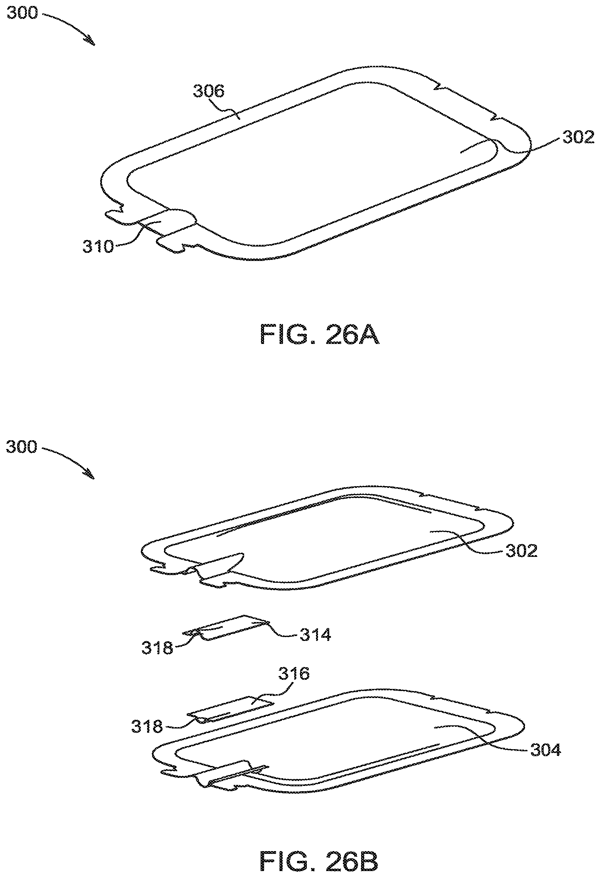

Referring now to FIG. 2, water from WFI vessel 200 must first be injected into pouch 300. To that end, first valve 142a is positioned to establish fluid communication between first syringe 120a and main water tube 202. Piston 122a associated with first syringe 120a is retracted proximally to draw a predetermined quantity of fluid from the WFI vessel 200 into first syringe 120a. First valve 142a is then positioned to establish fluid communication between first syringe 120a and contrast tube 206, as shown in FIG. 3. Piston 122a associated with first syringe 120a is advanced distally to inject a predetermined quantity of fluid, previously drawn into first syringe 120a from WFI vessel 200, into pouch 300 via contrast tube 206. As the fluid is injected into pouch 300, the fluid mixes with and reconstitutes the dry contrast media in the pouch 300 to create the reconstituted contrast media solution. Agitator 600 may be actuated during reconstitution of the contrast media to vibrate and heat pouch 300 and increase the efficacy (e.g. speed and homogeneity) of reconstituted contrast media. As the fluid is injected into pouch 300, the walls of pouch 300 may stretch to accommodate the increase in volume, as will be described in detail herein with reference to FIGS. 26A-28.

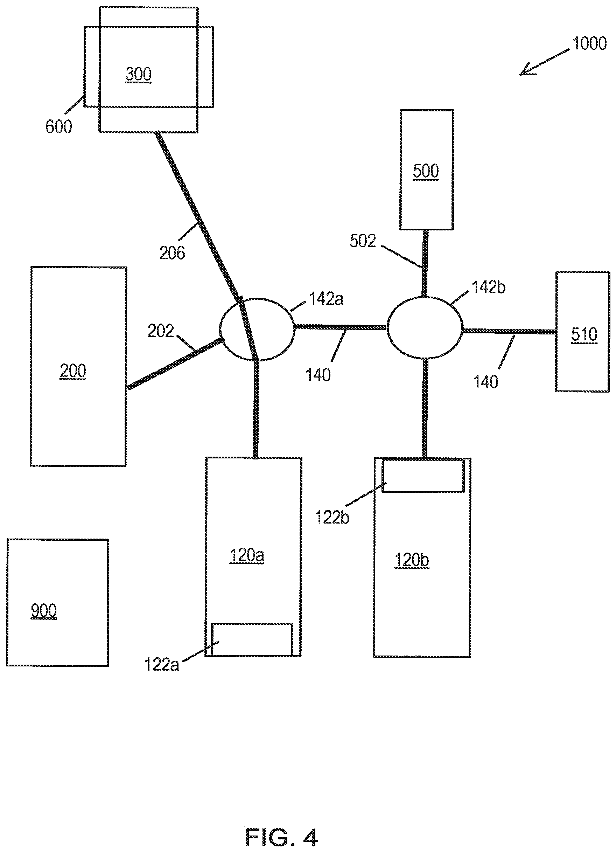

Once the contrast media in pouch 300 has been reconstituted, first valve 142a is maintained in the same position as in FIG. 3 to maintain fluid communication between first syringe 120a and contrast tube 206. As shown in FIG. 4, piston 122a associated with first syringe 120a may then be retracted to draw the reconstituted contrast media from pouch 300 into first syringe 120a.

At this stage, the reconstituted contrast media may be suitable for injection depending upon the status of the contrast media and whether it has been previously treated to remove particulates and/or impurities (e.g. endotoxins, dead viruses/bacteria), or whether the solute contrast media contains endotoxins or other impurities when container 300 is filled. In some non-limiting embodiments, container 300 may be heat treated such that the solute contrast media reaches a temperature of at least 150 degrees Celsius and such that the solute contrast media is sterilized prior to the agitator receiving the container. In some non-limiting embodiments, container 300 may be heat treated such that the solute contrast media reaches a temperature of 200 degrees Celsius and such that the solute contrast media is both sterilized and cleaned via depyrogenation prior to the agitator receiving the container. In some non-limiting embodiments, container 300 may be filled aseptically using non-pyrogenic (endotoxin free) solute contrast media.

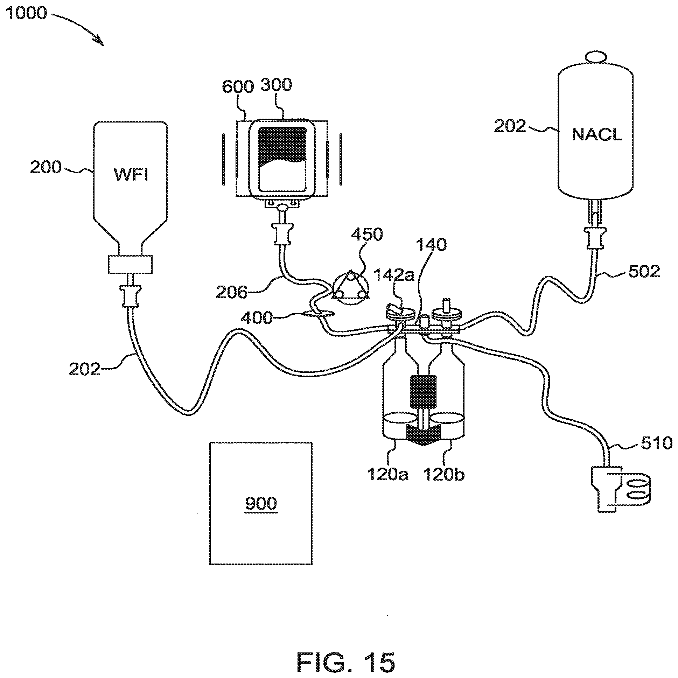

Where pouch 300 and the solute contrast media has previously been heat treated such that it has undergone depyrogenation (discussed in detail below with reference to FIGS. 26A-27C), the reconstituted contrast media in first syringe 120a is ready for safe delivery to administration line 510 and injection into the patient. However, where the solute contrast media has not been treated prior to reconstitution, or where further removal of particulates and/or impurities is indicated, then particulates/impurities will subsequently be removed from the reconstituted contrast media via filtration prior to delivery to the administration line 510 and injection into the patient. Filtration aspects of the present disclosure are described herein with reference to FIGS. 15-25.

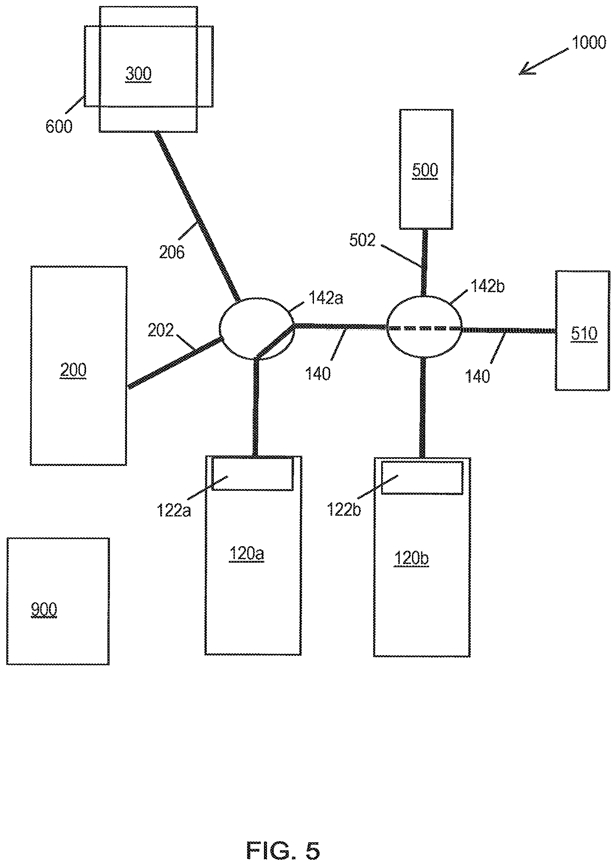

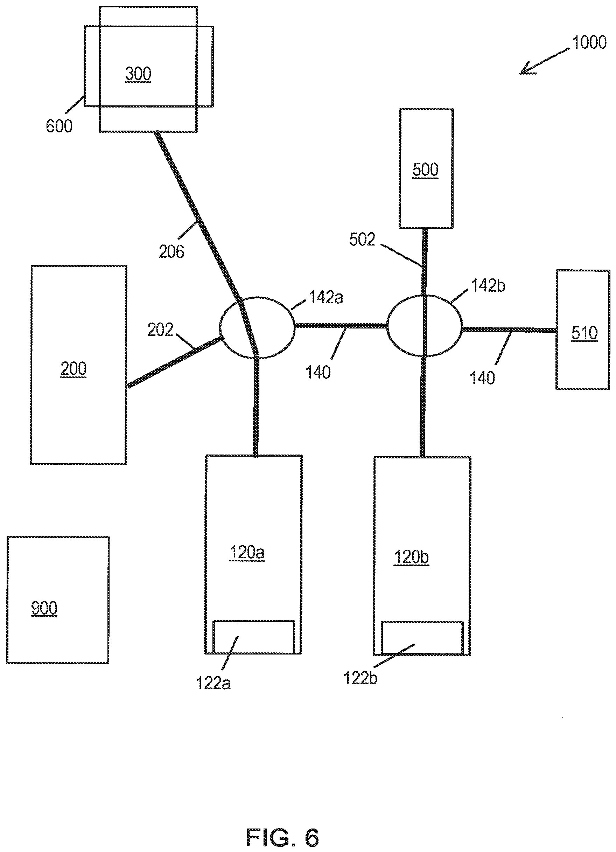

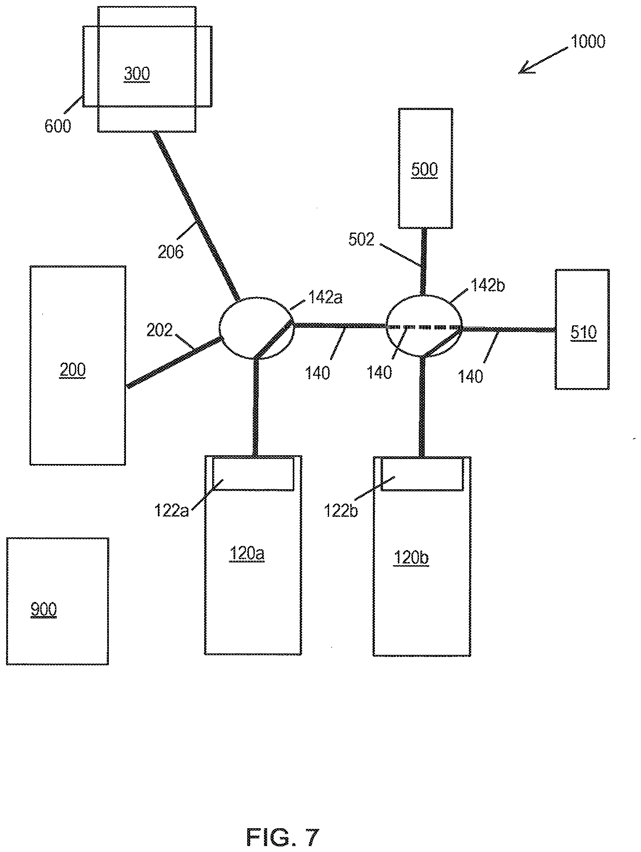

With the reconstituted contrast media loaded into first syringe 120a, first valve 142a may be positioned to establish fluid communication between first syringe 120a and the administration line 510, as shown in FIG. 5. First piston 122a associated with first syringe 120a may be advanced to inject the injectable reconstituted contrast media solution toward the administration line 510. As shown in FIG. 6, while the reconstituted contrast media is drawn into syringe 120a, valve 142b may be positioned to establish fluid communication between second syringe 120b and flushing agent tube 502. Second piston 122b associated with second syringe 120b may be retracted to draw the flushing agent into second syringe 142b. With the injectable reconstituted contrast media solution loaded into first syringe 120a and the flushing agent loaded into second syringe 120b, valves 142a, 142b may be positioned to establish fluid communication between syringes 120a, 120b and the administration line 510. First piston 122a and second piston 122b may then be advanced to inject the injectable reconstituted contrast media solution and the flushing agent (e.g. saline) into the administration line 510 and into the patient, as shown in FIG. 7.

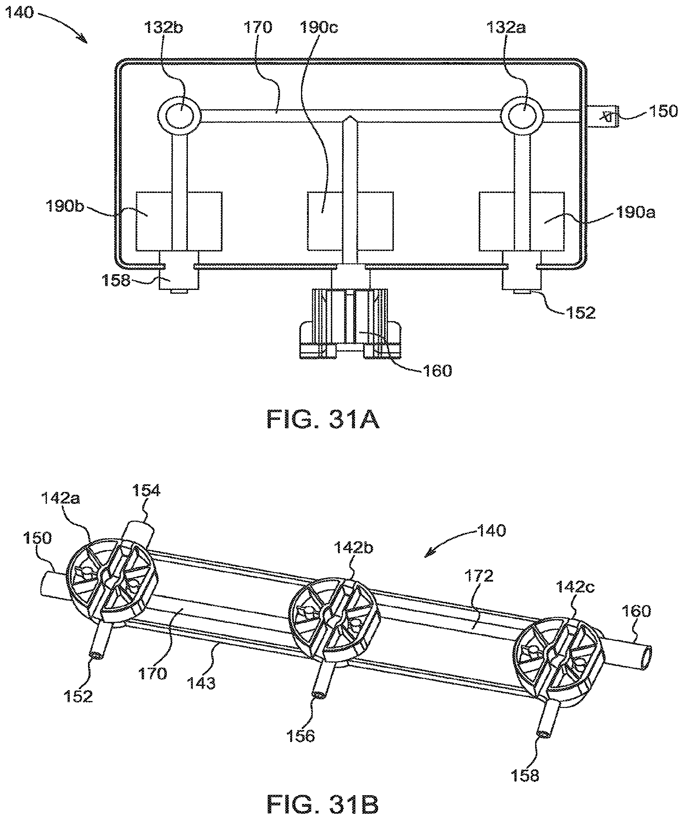

The use of dotted lines in FIGS. 5, 7, and 8 is used for illustration purposes and is demonstrative of the fluid communication between the syringes (e.g. first syringe 120a) and the administration line 510 via manifold 140. However, it is to be understood, that while the fluid communication between syringe 120a and administration line 510 may be established through one or more other valves (e.g. second valve 142b), the communication may also be established via directed communication through a conduit (e.g. conduit 170) of manifold 140, as described herein with references to FIGS. 31A and 31B.

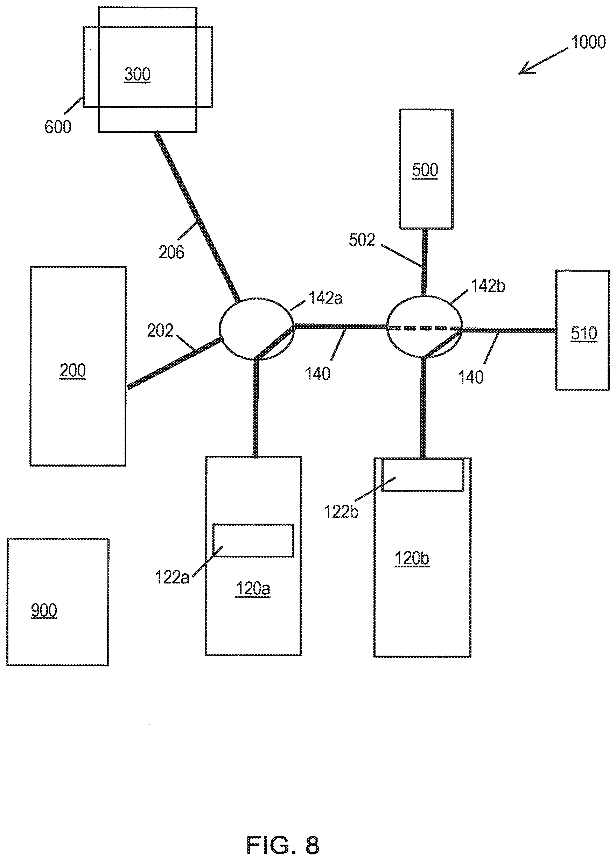

Some injection procedures may not require the entire volume of reconstituted contrast media generated from pouch 300. In such procedures, a surplus of liquid contrast media is present in first syringe 120a after the injection procedure is completed. This unused contrast media can be returned to pouch 300, in liquid form, and stored for recycling. With reference to FIGS. 8-9, controller 900 may be configured to deliver an unused portion of the injectable reconstituted contrast media solution from first syringe 120a to container 300. The process for retrieving unused contrast media is shown in FIGS. 8-9.

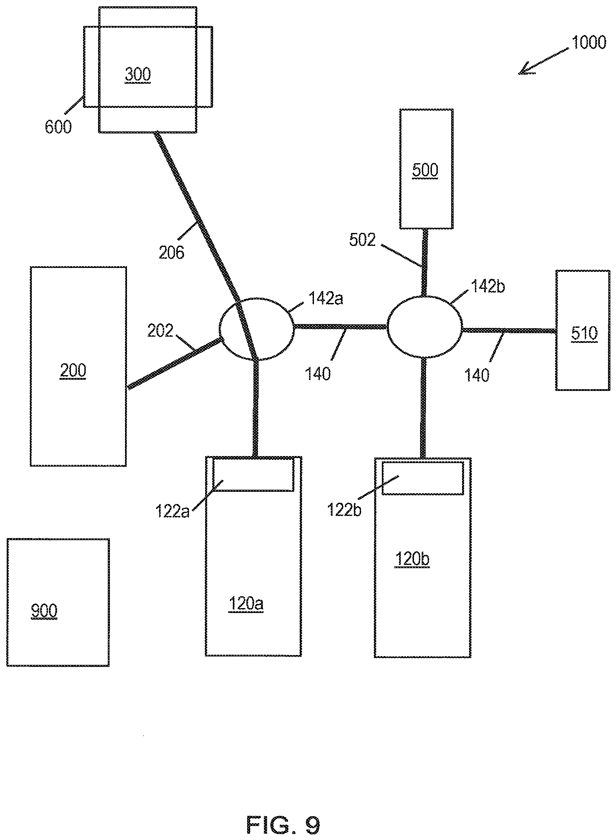

Referring to FIG. 8, first syringe 120a is partially filled with unused contrast media following an injection procedure. As such, piston 122a is in an intermediate position within the syringe 120a, with the volume between piston 122a and the distal end of syringe 120a filled with unused contrast media. As shown in FIG. 9, first valve 142a is positioned to establish fluid communication between first syringe 120a and pouch 300. Piston 122a is advanced to deliver the unused contrast media from the first syringe 120a into the pouch 300. Pouch 300 may then be disconnected from agitator 600 and from contrast line 206, and may undergo further recycling or reclamation processes.

Actuation of the valves 142a, 142b to the various positions as shown in FIGS. 2-9 may be automatically performed by controller 900 according to a pre-programmed injection procedure. Likewise, advancing/retracting pistons 122a, 122b as shown in FIGS. 2-9 may be automatically performed by the controller 900 according to a pre-programmed injection procedure.

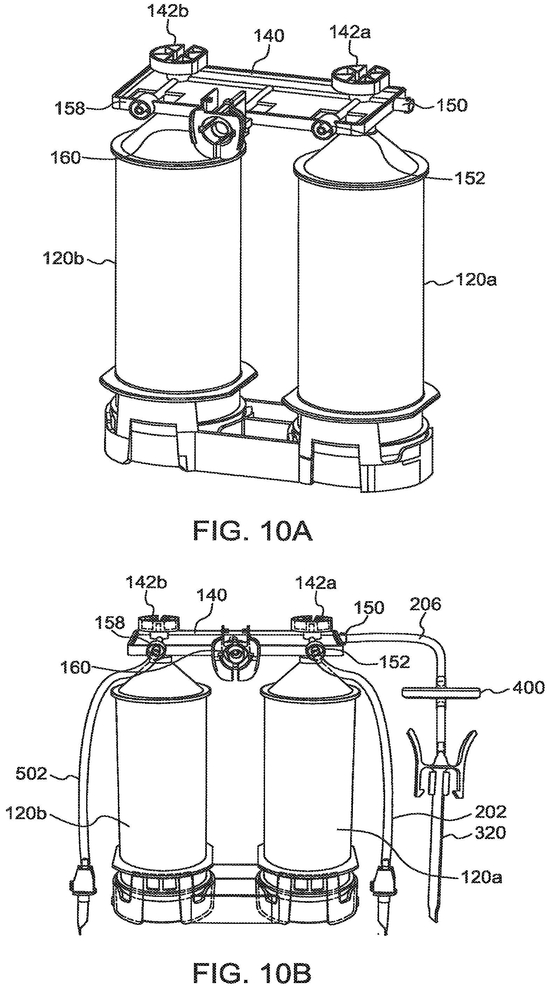

Having generally described a process of reconstituting and injecting contrast media from pouch 300, additional details of a non-limiting embodiment of fluid injector system 1000 having a two barrel fluid control configuration will now be described. Referring now to FIGS. 10A and 10B, FIG. 10A shows a non-limiting embodiment of syringes 120a, 120b, valves 142a, 142b and distribution manifold 140 of fluid injector system 1000. Valves 122a, 122b of system 1000 may be attached to an upper portion of manifold 140 while syringes 120a, 120b, may be connected to a lower portion of manifold 140 such that valves 142a, 142b control flow to and from syringes 120a, 120b. Distribution manifold may further include ports 150, 152, 158, 160 for fluid connection to various other components of system 1000. As shown in FIG. 10B, ports 150, 152, 158 may facilitate the fluid connection between syringes 120a, 120b and various lines 202, 206, 502. FIG. 10B also shows connection of contrast tube 206 with spike 320 for insertion into a container such as pouch 300. Further, FIG. 10B shows optional filtration module 400 for removing particulates and/or impurities from the reconstituted contrast media, as described below with references to FIGS. 15-25.

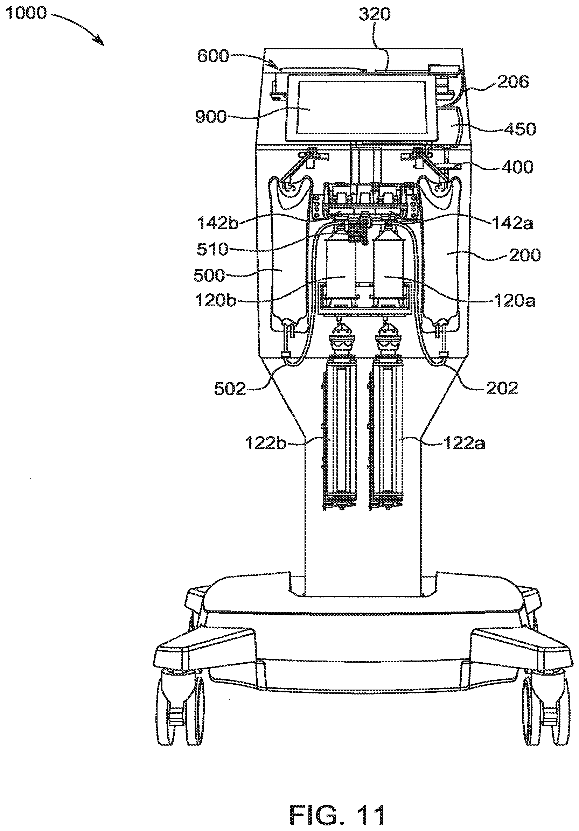

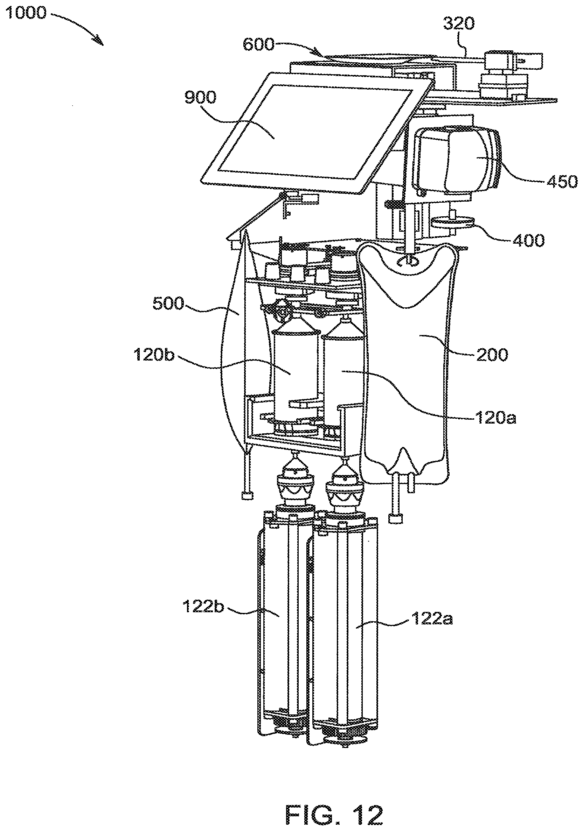

Referring now to FIGS. 11-15, the non-limiting embodiment of fluid injector system 1000 and a flow control assembly illustrated may be the same or similar to system 1000 shown and discussed with reference to FIGS. 1-9. In these embodiments, components of the flow control assembly are substantially similar to the embodiment of FIG. 1-9, with like numerals used to indicate like components. As shown, system 1000 may include syringes 120a, 120b, an administration line 510, a container 300 containing solute contrast media, valves 142a, 142b, agitator 600, and controller 900. As shown in FIG. 11, controller 900 may be a computer or tablet comprising at least one processor.

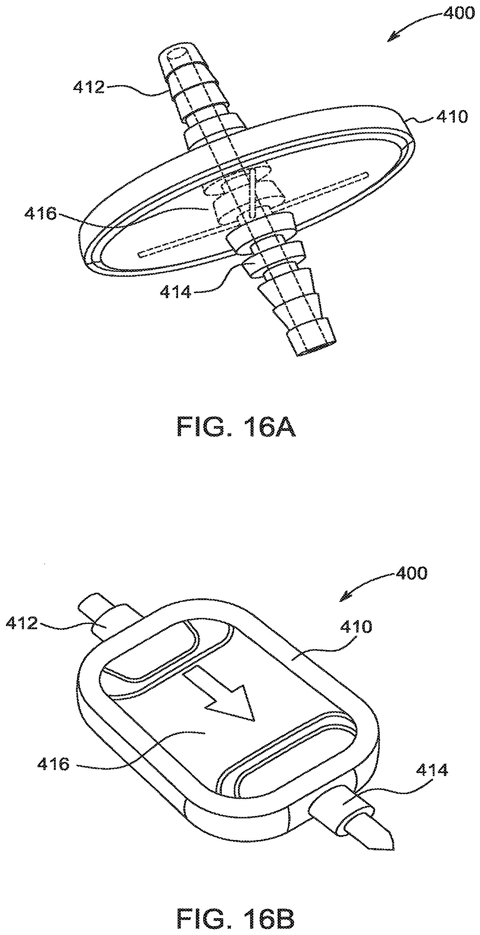

In the exemplary embodiment, system 1000 may include filtration module 400 (discussed below with reference to FIGS. 16A and 16B), between container 300 and first valve 142a. In some non-limiting embodiments, system 1000 may further include pump 450 configured to be in fluid communication with container 300, filtration module 400 and first syringe 142a. Filtration module 400 and pump 450 may be positioned between a spike 320 of contrast line 206 and first valve 142a as shown in FIG. 11. Filtration module 400 may be provided In some non-limiting embodiments, and may be configured to remove only coarse particles (e.g., particles of 10-20 microns or bigger) or only use a small pore filter to remove smaller particles that may also require use of a supplemental pump, such as pump 450, to help drive the fluid through such a filter. Other embodiments may not use a filtration module at all because dry heat sterilization may provide sufficient temperatures to make filtration unnecessary for removal of endotoxins.

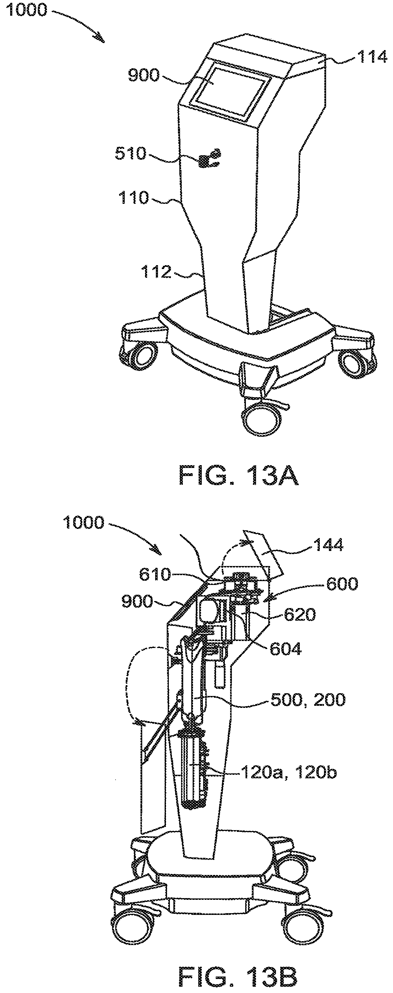

Referring now to FIGS. 13A and 13B, system 1000 may include housing 110 for containing and protecting the flow control assembly and other components of system 1000. System 1000 may further include stand 112 for mounting various components of system 1000. As shown in Fig. 13A, housing 110 may enclose the system such that only controller 900 is exposed so a user may monitor and control system 1000, and administration line 510 is exposed for connection to a patient.

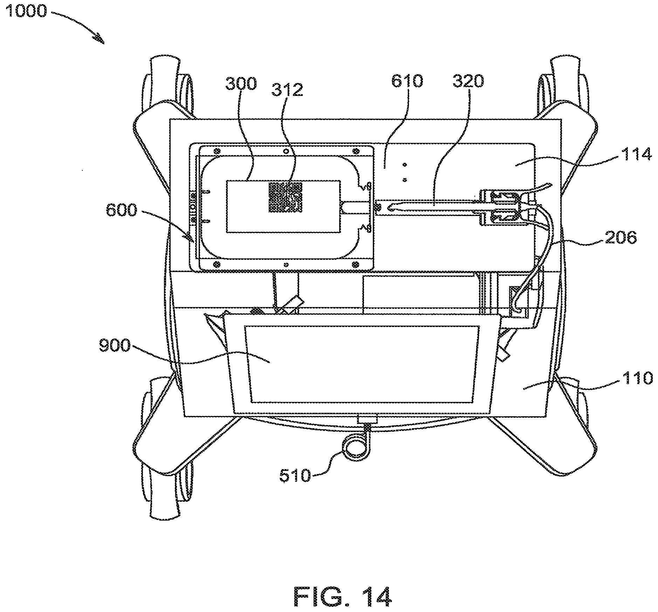

As shown in FIGS. 13A and 13B, housing 110 may have compartments such as lid 114 that open and close to expose components of system 1000 for set, maintenance, or other interaction with components of the flow control assembly of system 1000. For example, as shown in FIG. 14, lid 114 may be opened to allow access to agitator 600 and plate 610 to introduce container 300 to the system. Other openings may be provided to allow access to syringes, 120a, 120b; valves 142a, 142b; pistons 122a, 122b; diluent vessel 200; flushing agent vessel 500; water tube 202; flushing agent tube 502; etc. for setup or maintenance of system 1000.

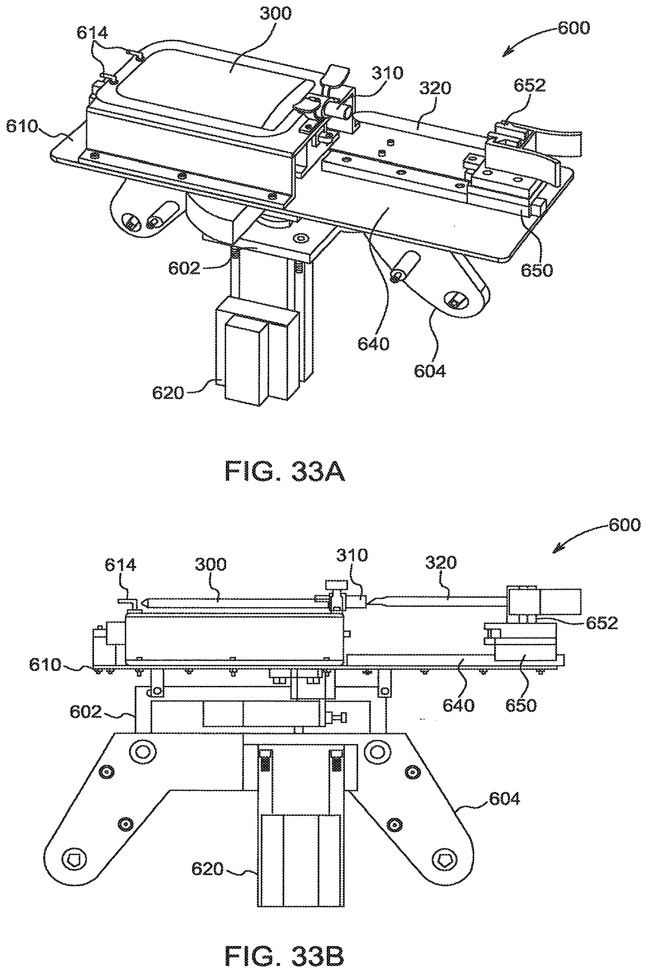

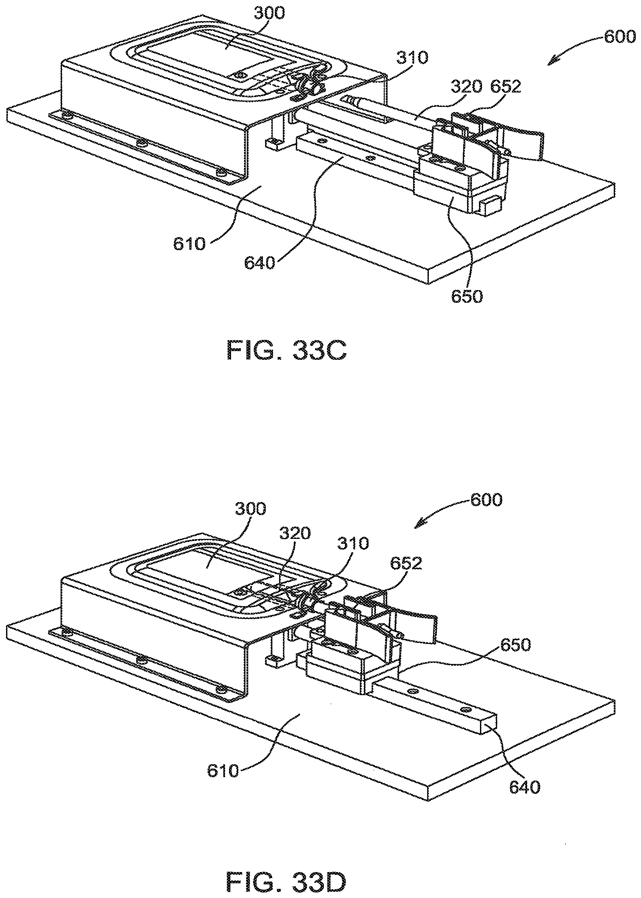

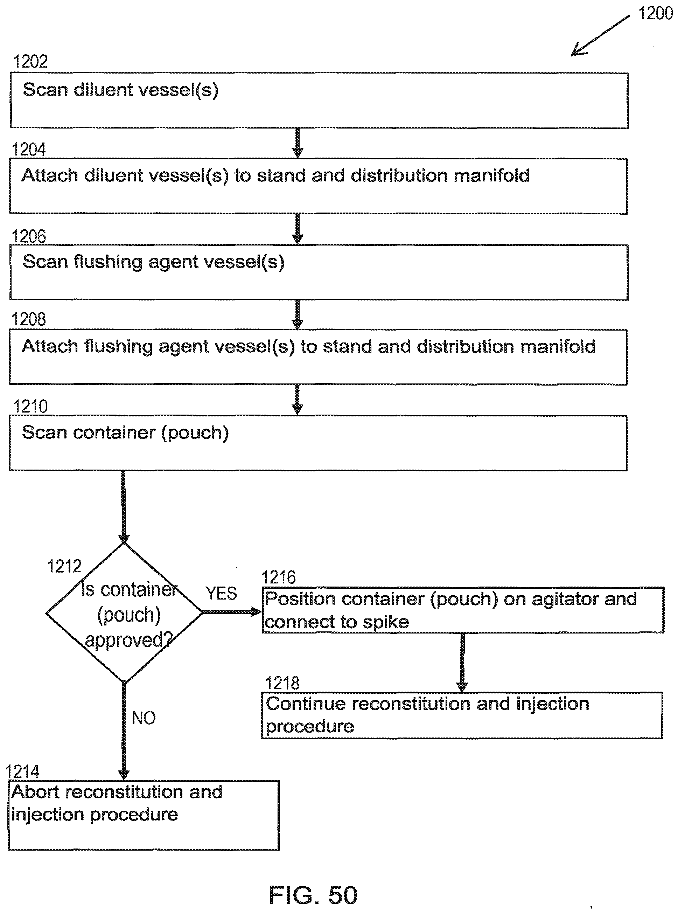

As shown in FIGS. 13B and 14, agitator 600 may be mounted to housing 110 or stand 112 of fluid injector system 1000 via a bracket 604. Agitator 600 may be configured to receive container 300 and may include moveable plate 610 to which container 300 may be secured, such that pouch 330 may be fluidly connected to contrast line 206 via spike 320 during reconstitution. Container 300 may be secured to plate 610 such that a solute contrast media identifier such as identifying indicia 312 is visible to a reader as discussed below with reference to FIG. 50. A motor (e.g. servo motor 620) may also be mounted to agitator 600 to facilitate agitation of plate 610 during reconstitution. Additional details of the exemplary embodiment of agitator 600 are shown and described below with reference to FIGS. 33A-33D.

Referring now to FIG 15, FIG. 15 is a schematic of a non-limiting embodiment of fluid injector system 1000 shown in FIGS. 11-14. In this exemplary embodiment, the fluid control assembly includes two barrels (syringes 120a, 120b) a filtration module 400, and a pump 450. Operation of the system shown in FIGS. 11-15 may be substantially the same or similar to that discussed with reference to FIGS. 1-9, with the addition of the optional filtration module 400 and pump 450. Accordingly, first valve 142a may be configured to provide selective fluid communication between one or more WFI vessels 200, first syringe 142a, filtration module 400, pump 450, and container 300. During reconstitution, after the diluent has been drawn from WFI vessel 200 into first syringe 120a and as WFI is being delivered to container 300, WFI passes through filtration module 400 and pump 450. Because the WFI is already ideally sterile, passing it though filtration module 400 to reach the solute contrast media contained in container 300 may have little to no effect on the WFI or on the filtration module. Similarly, as the WFI is ideally non-pyrogenetic, there is little or no resistance as the WFI passes through filtration module 400, and pump 450 may not be activated to deliver the WFI into container 300

Once the diluent/WFI is delivered to container 300, and the solute contrast media is reconstituted into a reconstituted contrast media solution, the contrast media is drawn into first syringe 120a via contrast tube 206 and valve 142a, and passes through pump 450 and filtration module 400. As discussed with reference to FIGS. 11-15, filtration module 400 may remove only coarse particles or may use a small pore filter to remove smaller particles. Removal of these particles may cause resistance as the reconstituted contrast media pass through filtration module 400, and may require use of pump 450 to help drive the reconstituted contrast media through the filter. After the reconstituted contrast media passes through pump 450 and filtration module 400 and is drawn into first syringe 120a by retracting piston 122a (aided by pump 450 driving the flow), the reconstituted contrast media is contained in the first syringe 120a in a suitable state for delivery to administration line 510, and ultimately, to the patient.

Referring now to FIGS. 16A and 16B, non-limiting embodiments of filtration module 400 in accordance with the present disclosure may include at least one filter media and may be configured to filter the reconstituted contrast media solution. Filtration module 400 may include one or more filter units configured to remove particulates, endotoxins, bacteria, viruses, and/or the like from the reconstituted contrast media.

Embodiments of filtration module 400 shown FIGS. 16A and 16B include single filter housing 410, though in some embodiments several filter housings 410 can be fluidly connected in series and/or parallel to improve filtration efficacy. In the embodiments shown in FIGS 16A and 16B, filter housing 410 includes inlet port 412 configured for connection to filtration inlet line 402, and outlet port 414 configured for connection to first filtration outlet line 404 (as shown in FIG. 17). Filter housing 410 contains filter media 416 selected to remove various contaminants from the reconstituted contrast media. The filter media 416 may be a porous structure, such as a polyethersulfone (PES) membrane, configured to trap certain impurities (including particulates and bacteria) greater than a predetermined size, such as 0.2 microns. Filter media 416 may further be configured to bind to certain impurities (such as endotoxins). Filtration module 400 may exhibit one or more modes of action such as those targeting the size, charge and/or affinity of different pathogens or molecules. The filter media 416 may be positively charged to remove the negatively charged particles (e.g., endotoxins).

Referring now to FIG. 17, FIG. 17 shows a schematic of a non-limiting embodiment of fluid injector system 1000 in accordance with the preset disclosure in a three barrel configuration. As shown in the illustrated embodiment, system 1000 may include a flow control assembly having third syringe 120c and corresponding third valve 142c such that the system includes three syringes 120a, 120b, 120c fluidly connected to distribution manifold 140. In this exemplary three barrel embodiment, filtration module 400 is included in system 1000, and first syringe 120a, second syringe 120b, and their respective associated components may be configured to work in association for the reconstitution of solute contrast media and filtration of reconstituted contrast media solution. The filtration module 400 may be the same or similar to the filtration module 400 discussed herein with reference to FIGS. 16A and 16B. However, it is contemplated that another suitable filter or filter module may be used.

The distribution manifold 140 may be fluidly connected to one or more diluent vessels such as WFI vessels 200 via first valve 142a and configured to provide selective fluid communication between one or more of the container (pouch 300), first syringe120a, a diluent vessel (WFI vessel(s) 200), and filtration module 400. The one or more WFI vessels 200 may be fluidly connected to first valve 142a via a main water tube 202. Branch water tube 204 may extend from main water tube 202 upstream of first valve 142a. Branch water tube 204 may be fluidly connected to fourth valve 144 that may be remotely located from distribution manifold 140. It is noted that branch water tube 204 and fourth valve 144 are necessary only in embodiments of the system 1000 that include a pre-made liquid contrast injection source, and thus branch water tube 204 and fourth valve 144 need not be present in embodiments that use only dry contrast media for injection. Pouch 300 may be received by agitator 600.

Filtration module 400 may be fluidly connected to first valve 142a via a filtration inlet tube 402, and to fourth valve 144 via first filtration outlet tube 404. Fourth valve 144 may also be fluidly connected to second valve 142b via a second filtration outlet tube 406. One or more flushing agent vessels 500 may be fluidly connected to third valve 142c of the distribution manifold 140 via a flushing agent tube 502.

In some embodiments, fourth valve 144 may be fluidly connected to liquid contrast media vessel 550 via bypass tube 552. Liquid contrast media vessel 550 may contain contrast media already in liquid form, and may be used during an injection procedure supplemental to or in place of the contrast media reconstituted in pouch 300. In other embodiments, liquid contrast media vessel 550, bypass tube 552, branch water line 204, and fourth valve 144 may be omitted; and first filtration outlet tube 404 may be connected to second valve 142b.

First valve 142a may facilitate selective fluid flow control from WFI vessel(s) 200, the pouch 300, and the first syringe 120a. Second valve 142b may facilitate selective fluid flow control from second syringe 120b and second filtration outlet tube 406. Third valve 142c may be configured to provide selective fluid communication between one or more of third syringe 120c, administration line 510, and flushing agent vessel(s) 500. Fourth valve 144 facilitates fluid flow from branch water line 204, first filtration outlet tube 404, second filtration outlet tube 406, and bypass tube 552.

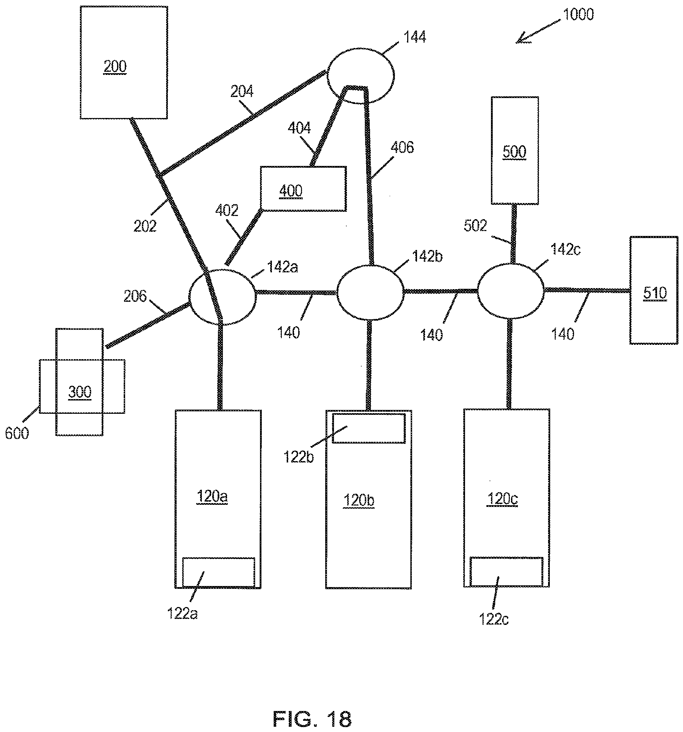

Controller 900 may be configured to actuate valves 142a, 142b, 142c, 144; pistons 122a, 122b, 122c associated with syringes 120a, 120b, 120c; and agitator 600 in in a prescribed sequences as shown in FIGS. 18-25 in order to reconstitute the solute contrast media contained in pouch 300 and ultimately deliver a dose to the patient.

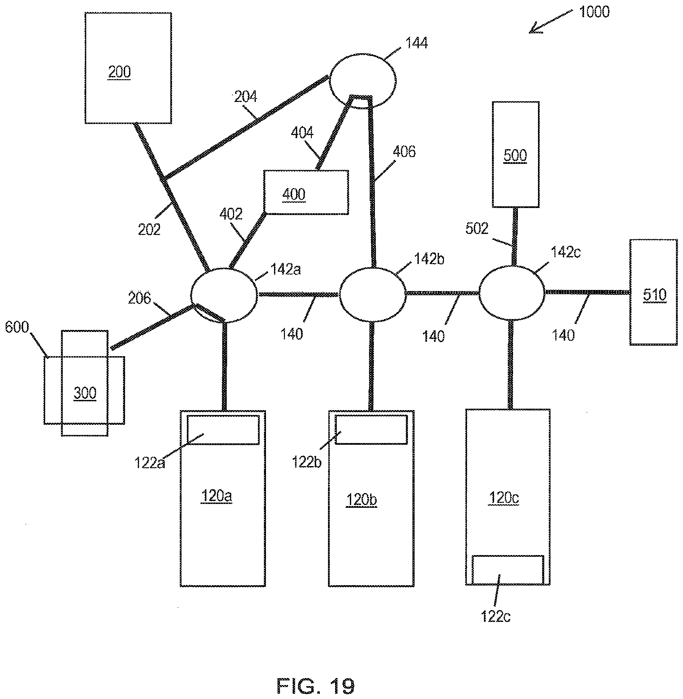

Referring now to FIG. 18, the diluent from WFI vessel(s) 200 must first be injected into pouch 300. To that end, first valve 142a may be positioned to establish fluid communication between first syringe 120a and main water tube 202. First piston 122a associated with first syringe 120a may be retracted proximally to draw fluid from WFI vessel(s) 200 into first syringe 120a. First valve 142a may then be positioned to establish fluid communication between first syringe 120a and contrast tube 206, as shown in FIG. 19. First piston 122a may be advanced distally to inject fluid from WFI vessel(s) 200, into pouch 300 via contrast tube 206. As the fluid is injected into pouch 300, the fluid mixes with and reconstitutes the solute contrast media in pouch 300 to create liquid contrast media. Agitator 600 may be actuated during reconstitution of the contrast media to agitate the pouch and increase the efficacy of the reconstituted contrast media.

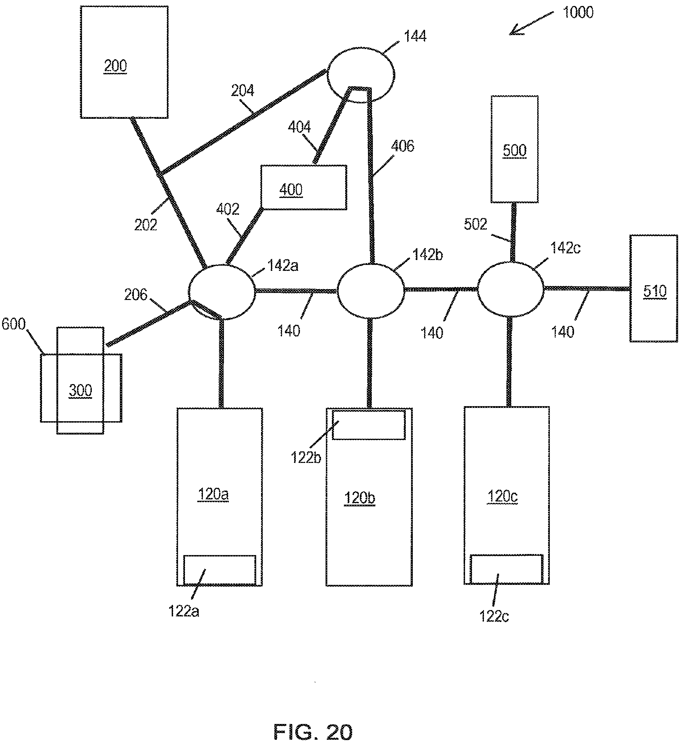

Once the contrast media in pouch 300 has been reconstituted, first valve 142a is maintained in the same position as in FIG. 19 to maintain fluid communication between first syringe 120a and contrast tube 206. As shown in FIG. 20, the piston 122a associated with first syringe 120a may then be retracted to draw the reconstituted contrast media from pouch 300 into first syringe 120a. At this stage, the reconstituted contrast media may contain particulates and/or impurities as discussed above, which will subsequently be removed via filtration. While the filtration module 400 is presently discussed in the context of a three barrel configuration as typically being located between first syringe 120a and second syringe 120b, this may not necessarily be the case in the two barrel configuration, as discussed above.

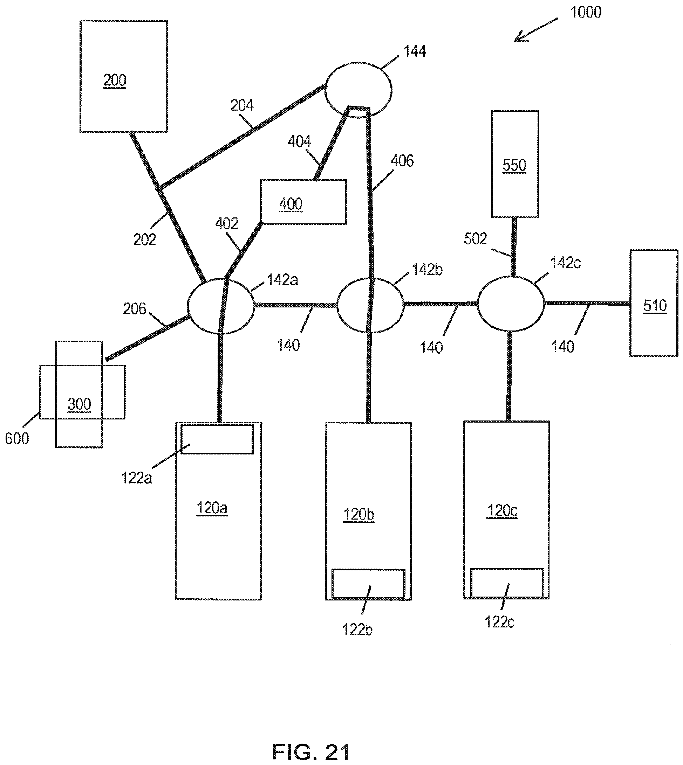

With the reconstituted contrast media loaded into first syringe 120a, first valve 142a may be positioned to establish fluid communication between first syringe 120a and filtration inlet tube 402, as shown in FIG. 21. Fourth valve 144 may be positioned to establish fluid communication between first filtration outlet tube 404 and second filtration outlet tube 406. Second valve 142b may be positioned to establish fluid communication between second syringe 120 and second filtration outlet tube 406. As such, a fluid path may be created from first syringe 120a, through filtration module 400, and into second syringe 120b. First piston 122a associated with first syringe 120a may be advanced to inject the reconstituted contrast media in first syringe 120a through filtration module 400 and toward second syringe 120b. Simultaneous with injection of the reconstituted contrast media from first syringe 120a, second piston 122b associated with second syringe 120b may be retracted to draw the reconstituted contrast media into second syringe 120b. As the reconstituted contrast media passes through filtration module 400 on route to second syringe 120b, filtration module 400 removes any particulates and impurities from the reconstituted contrast media. Thus, the reconstituted contrast media loaded in second syringe 120b is suitable for injection into the patient. The filtration process may take approximately one minute in some embodiments, although the filtration time will be dictated by the size and density of the filter media of the filtration module, along with other factors such as the flow rate of the reconstituted contrast media through the filtration module. The filtration time is also predicated on (i) the number of particulates in the pouch and (ii) the number of injection cycles to which the filtration module has been exposed (e.g., injection n100 will require more time than injection n1 because of the particulates that have accumulated in the filtration module.)

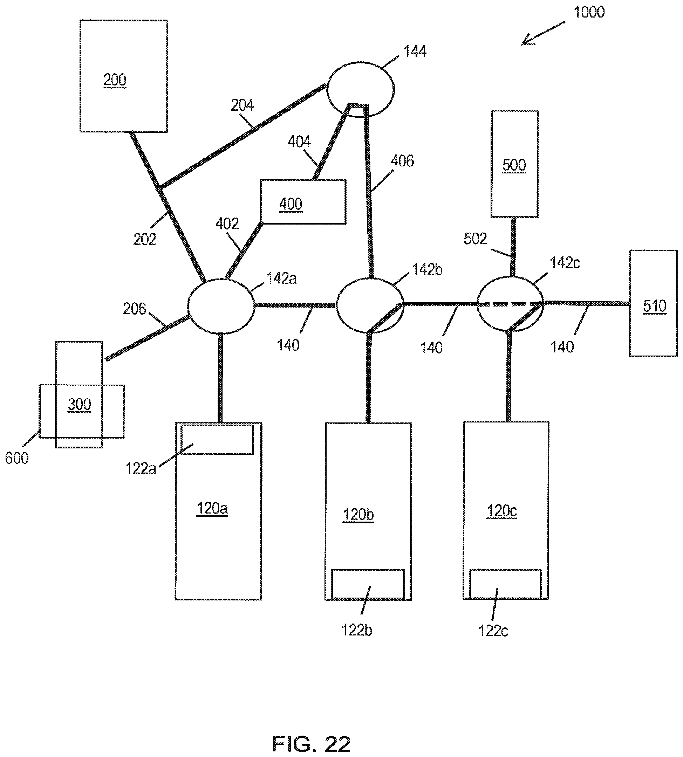

To initiate an injection procedure, second valve 142b is positioned to establish fluid communication with infusion set 510 via third valve 142c, as shown in FIG. 22. As such, advancement of second piston 122b associated with second syringe 120b may inject the reconstituted contrast media from second syringe 120b through second valve 142b, through the third valve 142c, into infusion set 510, and ultimately to the patient. In addition, this position of third valve 142c may also establish fluid communication between third syringe 120c and infusion set 510, allowing injection of a flushing agent (e.g. saline) from the third syringe 120c into the patient.

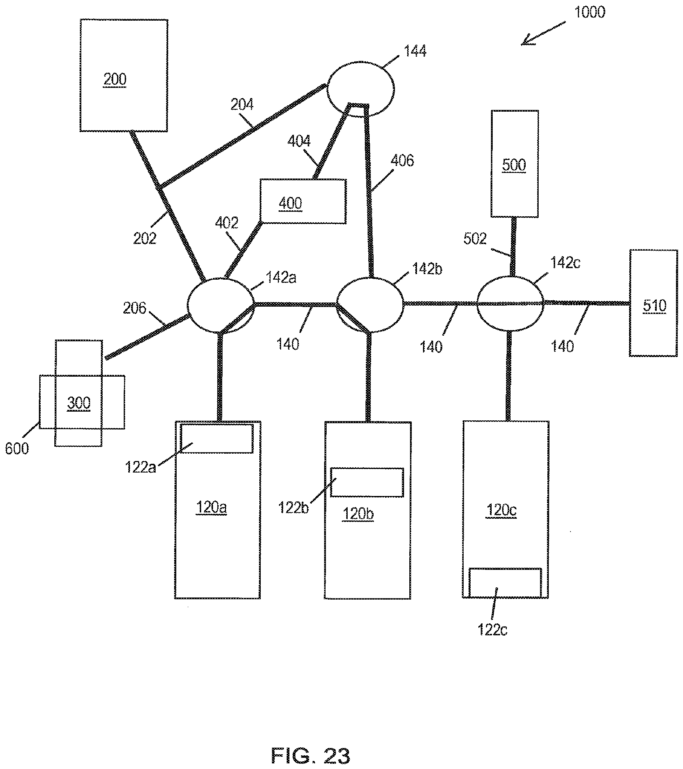

Referring to FIG. 23, second syringe 120b is partially filled with unused contrast media following an injection procedure. As such, second piston 122b is at an intermediate position within syringe 120b, with the volume between piston 122b and the distal end of syringe 120b filled with unused contrast media. As shown in FIG. 23, first valve 142a is positioned to establish fluid communication between first syringe 120a and second valve 142b. Second valve 142b is positioned to establish fluid communication between second syringe 120b and first valve 142a. As such, a flow path is created between first syringe 120a and second syringe 120b.

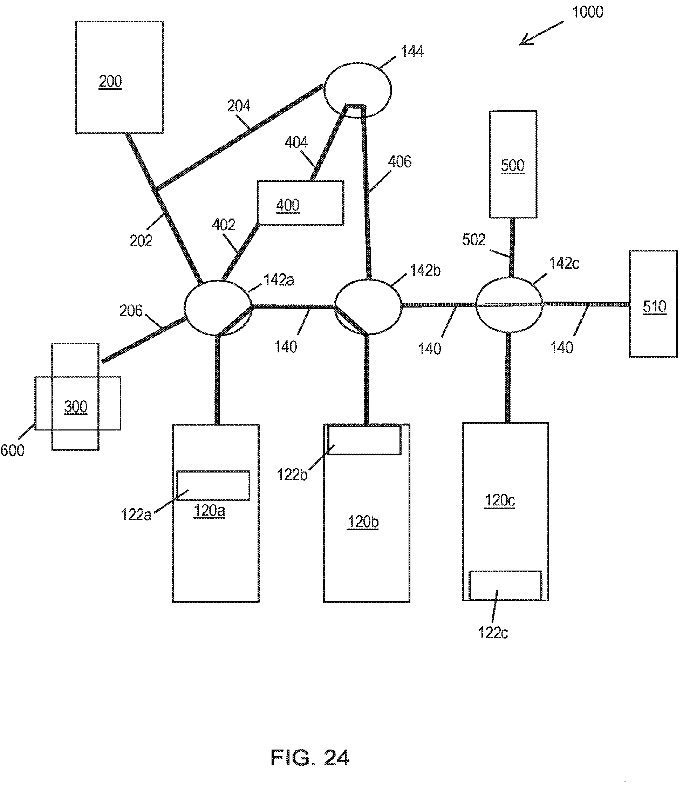

Referring now to FIG. 24, first piston 122a is retracted simultaneously with second piston 122b being advanced to inject the unused contrast media from second syringe 120b into first syringe 120a.

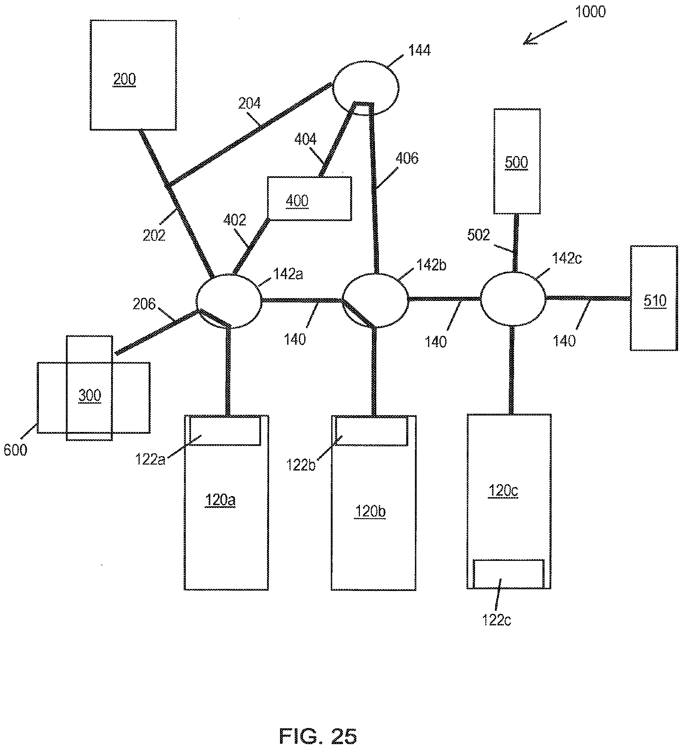

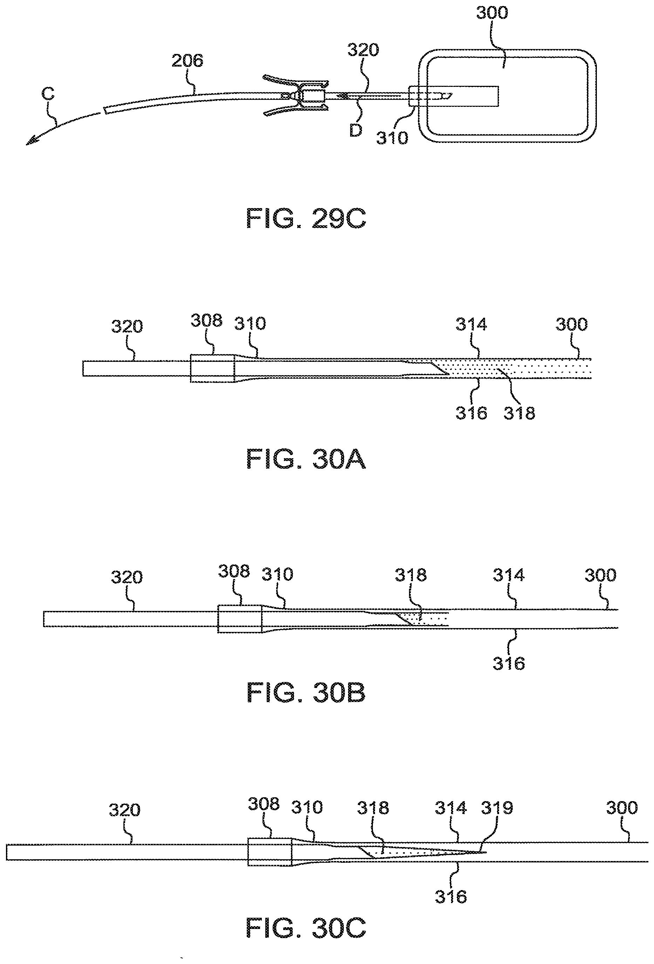

Referring now to FIG. 25, first valve 142a is positioned to establish fluid communication between first syringe 120a and pouch 300. First piston 122a can then be advanced to inject the unused contrast media from first syringe 120a into pouch 300. Pouch 300 may then be disconnected from agitator 600 and from contrast line 206, as shown in FIGS. 29C and 30C. Pouch 300, now filled with unused contrast media, may be sent and/or transported to an appropriate facility for recycling and/or reclaiming. In particular, the iodine and/or other active ingredients may be reclaimed from the unused contrast media. This eliminates the need for hazardous disposal of the unused contrast media.

As discussed above with reference to a non-limiting embodiment of system 1000 having two barrels, actuation of the valves 142a, 142b, 142c, 144 and advancing/retracting the pistons 122a, 122b, 122c to the various positions shown in FIGS. 18-25 may be automatically performed by the controller 900 according to a pre-programmed injection procedure. Accordingly, controller 900 may be programmed or configured to deliver the reconstituted contrast media solution from the container (e.g. pouch 300) to first syringe 120a, from first syringe 120a to filtration module 400, from filtration module 400 to second syringe120b, and from second syringe 120b to administration line 510. Further, controller 900 may be programmed or configured to deliver the flushing agent from flushing agent vessel 500 into third syringe 120c, and deliver the flushing agent from third syringe 120c to administration line 510.

Having generally described the process of reconstituting and injecting contrast media from pouch 300, additional details of a suitable container for containing and reconstituting solute contrast media, such as pouch 300, will now be described. Referring to FIGS. 26A-27C, pouch 300 may include a flexible membrane including a top membrane 302 and bottom membrane 304 joined at perimeter seam 306. Membranes 302, 304 may be sized to contain a predetermined quantity of dry contrast media between them. In some embodiments, pouch 300 may be configured to contain approximately 61.3 grams to approximately 63.3 grams of dry contrast media, and membranes 302, 304 may have a length and width of approximately 120mm and 85mm, respectively, in an area bounded by the perimeter seam 306. The amount of dry contrast media contained in pouch 300 between the membranes 302, 304 may be selected to be approximately equivalent to a standard dosage of conventional liquid contrast media. For example, 62.3 grams of dry contrast media may create a dosage, when reconstituted, approximately equal to 100 milliliters of conventional liquid contrast media. The powder formulation is designed so that the reconstituted contrast medium of the present disclosure will be equivalent to approved presentations.

When pouch 300 is empty, membranes 302, 304 may sit substantially flush with one another, as shown in FIGS. 26A and 27A, such that pouch 300 defines a negligible internal volume. The flexible membrane including membranes 302, 304 may be flexible, stretchable, and configured to expand so that as pouch 300 is filled with dry contrast media and fluid, membranes 302, 304 stretch to provide an interior volume for the reconstituting contrast media. Membranes 302, 304 may also be resilient so that after the contrast media is withdrawn from pouch 300, membranes 302, 304 return to the unstretched state of FIG. 27A.

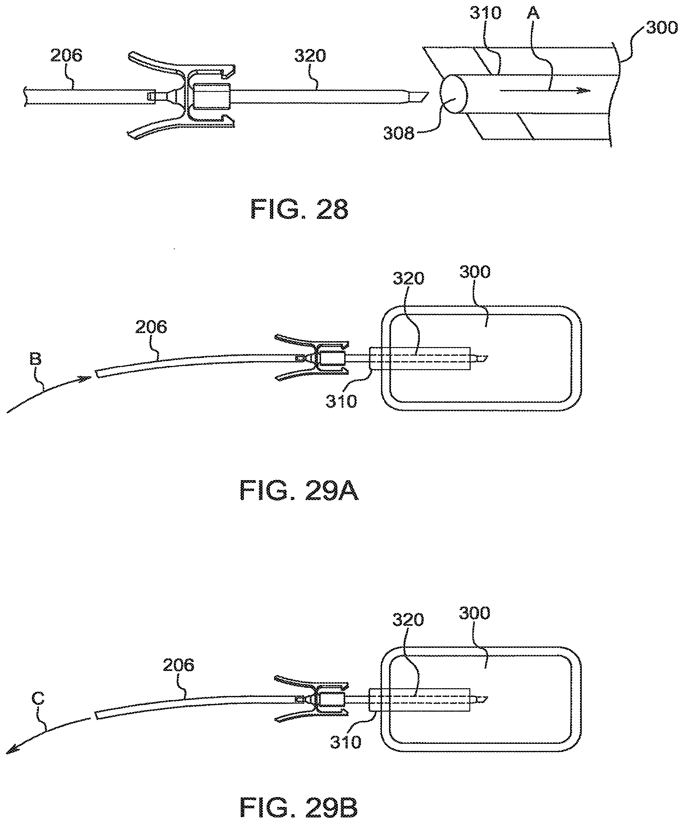

Internal valve 310 may be provided in pouch 300 and is configured to receive a spike of a contrast line to allow flow into and out of the internal volume between membranes 302, 304, such that it is in fluid communication with first valve 142a upon entry of the container into fluid injector system 1000. In some embodiments, as shown in FIG. 28, internal valve 310 defines orifice 308 through which a spike may be inserted. In some embodiments, internal valve 310 may be a film valve including opposing film portions 314, 316 that may be separated by the spike to access the interior volume of pouch 300 between membranes 302, 304. Internal valve 310 may be further configured to passively seal the container when the spike is removed from internal valve 310.

With reference to FIG. 27A, top and/or bottom membrane 302, 304 may include a readable solute contrast media identifier such as identifying indicia 312. Identifying indicia 112 may be a barcode, QR code, RFID tag, text, symbol, or the like that controller 900 or an operator may use to validate that an approved and correct pouch 300 has been connected prior to initiating reconstitution and patient injection.

Pouch 300 may be filled with dry contrast media as part of the manufacturing process. Any type of dry contrast media suitable for reconstitution may be contained in the pouch. In one example, the dry contrast media includes lopromide as the single active ingredient. The dry contrast media include the active ingredient as well as a complexing agent, a buffer, a pH adjustment agent, and combinations thereof. An example of the complexing agent can include sodium calcium edetate. An example of the buffer can be trometamol. An example of the pH adjustment agent can be a dry powder equivalent to hydrochloric acid (e.g. hydrochloric acid 10%), which is used to maintain an approximately neutral pH of the contrast media. The amounts of each ingredient can be configured so that the amounts match the ingredients of a conventional liquefied contrast media when accounting for WFI added to the dry contrast media during reconstitution. Other examples of suitable dry contrast media include but are not limited to: lohexol (trade name Omnipaque^{™}), lopamidol (trade name Isovue^{®}), lomeprol (trade name Iomeron^{®}), loversol (trade name Optiray^{®}), lobitridol (trade name Xenetix^{®}), or lodixanol (trade name Visipaque^{™}).

Pouch 300 may be sterilized prior to use in system 1000 in order to kill any contaminants such as viruses, bacteria, and other germs present in the dry contrast media. In some non-limiting embodiments, the dead contaminants may be subsequently filtered out by a filter such as filtration module 400 (see, e.g., FIGS. 15-22) to prevent inadvertent injection into the patient. However, as previously alluded to, filtering and/or the filtration module 400 are not required where the solute contrast media contained in the container undergoes high heat sterilization and/or depyrogenation prior to entering system 1000. Sterilization of pouches 300 may include electron-beam irradiation, gamma irradiation, low temperature dry heat sterilization processes, and combinations thereof. Sterilization may be performed on the pouches 300 individually, or on packaging containing a plurality of the pouches 300. The membranes 302, 304 must be made of a material that can withstand the sterilization process without degradation. For example, the flexible membrane and membranes 302, 304 may be made of a polymer, such as a recyclable plastic. The use of recyclable plastic also allows the pouch 300 to be recycled after use through conventional channels not involving hazardous material handling.

In some non-limiting embodiments, the material may be comprised of polyethylene terephthalate (PET) or polyether ether ketone (PEEK) to facilitate use of high temperature dry heat sterilization such that the solute contrast media undergoes depyrogenation. Other suitable polymeric materials may also be utilized so long as that material can sufficiently withstand the high temperature dry heat sterilization conditions for sterilization of the pouch and its contents, and avoid significant degradation after exposure to the sterilization heat for the sterilization time period that is used.

In some non-limiting embodiments, the use of high temperature dry heat sterilization obviates the need to filter the reconstituted contrast media solution such as with filtration module 400. High temperature dry heat sterilization involves heating of the container (pouch) 300 and its contents to at least 150 degrees Celsius, ideally to 200 degrees Celsius or over 200 degrees Celsius. The dry heat is applied to the container and its contents for a pre-selected dry heat sterilization time period, (e.g., 1 hour, 1.5 hours, etc.) such that the solute contrast media contained within the container 300 is sterilized.. This sterilization process can sterilize the solute contrast media and container 300 so any contaminants such as viruses, bacteria, or other pathogens are dead. When high temperature dry heat sterilization is used, it is contemplated that the reconstituted contrast media can be passed through for injection into a patient without use of filtering as the higher temperature also breaks down endotoxins resulting in a pyrogen free material. The temperature higher temperature required to break down endotoxins is 200 degrees Celsius or over 200 degrees Celsius. Accordingly, container or pouch 300 may be heat treated such that the solute contrast media reaches a temperature of at least 150 degrees Celsius and such that the solute contrast media is sterilized, or preferably at least 200 degrees Celsius, such that the solute contrast media is cleaned via depyrogenation.

Alternatively, a filtration module 400 can be used during the injection process that only utilizes coarse particle filtering (e.g. removal of larger particulates) or uses another type of filter or filtration module that is configured to remove particulates that are 0.22 microns or between 0.22 microns and 0.10 microns (e.g. filtration for removal of endotoxins, and dead bacteria can be removed via filtration which can be about 0.22 microns in size). It is possible that dead viruses that may be too small to filter may pass into the patient via injection as dead viruses. If this were to occur, this is believed to pose no significant health risk to a patient as the viruses are dead.

Accordingly, there are various methods associated with the manufacture and preparation of the solute contrast media to be sealed in container 300 and the container 300 for use with the methods and the fluid injector systems of the present disclosure. The preparation of the solute contrast media and container 300, in part, may dictate the method of reconstitution.

In some non-limiting embodiments, a method involving use of a solute contrast media in which the presence of endotoxins is possible or uncertain may include: filling the container 300 (e.g., pouch) with a solute contrast media in which endotoxins may be present; heating the container 300 with the solute contrast media therein to a temperature of at least 150 degrees Celsius; reconstituting the solute contrast media with WFI to form a reconstituted contrast media solution; and filtering the reconstituted contrast media solution to remove therefrom: coarse particles via a filter designed to trap particles of 10-20 microns or larger, or smaller particles via a small pore filter designed to trap particles greater than a predetermined size (e.g., 0.2 mm) wherein the filtration may, as an option, be aided with the use of a supplemental pump to help drive the solution through the small pore filter. The filter used in this filtration step is intended to remove particulates, endotoxins, bacteria, viruses, and/or the like from the reconstituted contrast media solution.

In other non-limiting embodiments a method involving use of a solute contrast media in which the presence of endotoxins is possible or uncertain may include: filling the container 300 (e.g., pouch) with a solute contrast media in which endotoxins may be present; heating the container 300 with the solute contrast media therein to a temperature of at least 200 degrees Celsius for a preselected dry heat sterilization time period; and reconstituting the solute contrast media with WFI to form a reconstituted contrast media solution. There is no need to employ a filter in this method because the higher temperature (i.e., 200 degrees Celsius or over) will kill bacteria, viruses and other pathogens and break down the endotoxins released therefrom resulting in a pyrogen free material.

In other non-limiting embodiments, a method involving the production of the solute contrast media as a non-pyrogenic (endotoxin free) powder that is sealed within the container in a sterile environment includes: providing a sterile environment; manufacturing the solute contrast media in the sterile environment so that the solute contrast media is produced as a non-pyrogenic (endotoxin free) powder; aseptically filling the container 300 with the non-pyrogenic (endotoxin free) powder in the sterile environment thereby producing a pre-sterilized pouch containing a pre-sterilized solute contrast media such that the pre-sterilized pouch is ready for use in the methods and fluid injector systems of the present disclosure without the need for the heating and filtration steps required in other non-limiting embodiments.

After sterilization, the sterilized pouches can be placed into a box or other container for transport to a care facility. Alternatively, pouches can be positioned in packing or a box and subsequently undergo the dry heat sterilization for a pre-selected sterilization time to sterilize the pouches before the box is transported to a care facility.

Use of system 1000 for reconstituting the contents of a container such as pouch 300 may provide significant sustainability improvements including increased packaging efficiency, significant reductions in emissions and energy consumption, and recyclability of components. For example, pouch 300 allows more doses to be shipped and stored in the same volume of packaging as traditional liquid contrast media. The pouches also require less energy to manufacture than traditional contrast media vessels. Accordingly, CO2 emissions, energy to manufacture, shipping mass, and shipping volume are all reduced, decreasing environmental impact and cost. The pouches are fully recyclable via conventional recycling streams, and solutes such as iodine may be recovered from unused contrast media. Therefore, there are significant improvements associated with cost and environmental impact provided by implementation of system 1000 and the associated devices and methods discussed herein.

Referring now to FIG. 28, once sterilized (and/or cleaned via depyrogenation) and removed from any packaging, pouch 300 may be connected to contrast tube 206. Contrast tube 206 may include spike 320 for connection to internal valve 310 of pouch 300. To connect pouch 300 to contrast tube 206, spike 320 may be inserted into valve 310 in direction A to open orifice 308 and establish fluid communication between the contrast tube 206 and the interior of the pouch 300 containing the dry contrast media. Once this connection is made, the dry contrast media can be reconstituted, as shown in FIG. 29A, by injecting fluid through contrast tube 206 in direction B into pouch 300. This injection can be achieved by appropriate actuation of first valve 142a and piston 122a (as shown in FIGS. 3 and 19). As shown in FIG. 29B, the reconstituted liquid contrast media can be withdrawn from pouch 300 via contrast tube 206 by creating a vacuum in the direction C, such as by retracting piston 122a (as shown in FIGS. 4 and 20). FIG. 29C illustrates disconnection of pouch 300 from contrast line 206. Spike 320 is withdrawn from valve 310 in direction D. Concurrently, a vacuum is applied to contrast tube 206 in direction C (for example by retracting the piston 122a of FIG. 4) to reseal internal valve 310 while ensuring no contaminates are able to enter.

Referring now to FIGS. 30A-30C, additional details of spike 320 being inserted into and removed from pouch 300 are shown. Referring first to FIG. 30A, valve 310 may include upper film 314 and lower film 316 that are spread apart by spike 320 to define inner conduit 318. With spike 320 fully inserted into internal valve 310, as shown in FIG. 29A, inner conduit 318 extends into the inner cavity of pouch 300 to allow flow of fluid from spike 320 into pouch 300 and vice versa. Referring flow to FIG. 30B, with spike 320 partially inserted into (or partially removed from) internal valve 310, upper and lower films 314, 316 passively relax towards each other, reducing the cross sectional area of inner conduit 318. At a certain depth of insertion of spike 320, upper and lower films 314, 316 relax such that upper film 314 meets lower film 316 at point 318, as shown in FIG. 29C. At point 319, inner conduit 318 is closed such that no fluid can occur into or out of internal valve 310, as shown in FIG. 30C. The passive seal created at point 319 allows spike 320 to be fully removed without the contents of pouch 300 leaking out, and without any contaminants from the environment entering pouch 300.

Referring now to FIGS. 31A and 31B, non-limiting embodiments of distribution manifold 140 of system 1000 is shown in detail. FIG. 31A shows an exemplary distribution manifold 140 configured for a two barrel configuration of system 1000 such as system 1000 shown in FIGS. 1-9. FIG. 31B shows an exemplary distribution manifold 140 configured for a three barrel configuration of system 1000 such as system 1000 shown in FIGS 17-25. Distribution manifold 140 may have different ports and features depending on the embodiment or configuration of system 1000. In some non-limiting embodiments, distribution manifold 140 includes a main body 143 to which valves 142a, 142b, etc. are connected via valve seats 132a, 132b, etc. Distribution manifold 140 may include contrast port 150, WFI port 152, flushing agent port 158, and an infusion port 160 configured for connection to an infusion set such as administration line 510. Distribution manifold 140 may further include conduit, such as first manifold conduit 170 for fluidly connecting valves 142a, 142b, etc. and ports 150, 152, 158, 160, etc. within distribution manifold 140.

In some non-limiting embodiments, as shown in FIG. 31A, distribution manifold 140 may further include air detection windows 190a-190c to allow various sensors to detect various properties of fluids, such as the presence of air, as they enter and exit distribution manifold 140. As further shown in FIG. 31A, first manifold conduit 170 may not only fluidly connect first valve 142a and second valves 142b to each other, but may also fluidly connect valves 142a, 142b to infusion port 160 such that fluid passing through first valve 142a from first syringe 142a need not pass through second valve 142b, and fluid passing through second valve 142b from second syringe 120b need not pass through first valve 120a to reach infusion port 160. Valves 142a, 142b may be, for example, a stopcock 142 that can be rotated to change which of the ports 150, 152, 158, 160 and/or first conduit 170, are in fluid communication with syringes 120a, 120b and/or infusion set 510.

In some non-limiting embodiments, as shown in FIG. 31B, distribution manifold 140 may further include filtration inlet port 154 and filtration outlet port 156 to allow for filtering as described with reference to FIG 21. As shown in FIG. 31B, first manifold conduit 170 fluidly connects first valve 142a to second valve 142b, and second manifold conduit 172 fluidly connects second valve 142b to third valve 142c and thus infusion port 160. Valves 142a-142c may be, for example, a stopcock 142 that can be rotated to change which of the ports 150, 152, 154, 156, 158, 160 and/or first and second conduits 170, 172 are in fluid communication with syringes 120a-120c and/or infusion set 510.

Valves 142a, 142b, 142c may be rotated to various positions, such as those shown in FIGS. 1-9 and 17-25, by controller 900 to dictate fluid communication between syringes 120a-120c; ports 150, 152, 154, 156, 158, 160; and first and second manifold conduits 170, 172. A non-limiting embodiment of stopcock 142 in accordance with present disclosure is shown in FIGS. 32A and 32B. Stopcock 142 may include fluid passage 146 and fluid channel 148. The direction and changing of the direction of fluid passage 146 may control which components of the system 1000 are fluidly connected. Stopcock 142 may further include stopcock drive engagement 132 by which system 1000 and controller 900 impart force via a motor or another like mechanism to change the direction of fluid passage 146 and thus fluid connection of various components of system 1000.

Referring now to FIGS. 33A-37B, agitator 600 includes base 602 that mounts to housing 110 or stand 112 of fluid injector system 1000 via bracket 604. Agitator 600 further includes moving plate 610 on which pouch 300 is removably mounted. Movable plate 610 is configured with at least one degree of freedom, and as such may translate along a plane and/or rotate about an axis. In some non-limiting embodiments, plate 610 both rotates about an axis perpendicular to plate 610, and translates along a plane parallel to plate 610. For example, plate 610 may be agitated by rotation, vibration, gyration, linear actuation, and/or other movements. In some non-limiting embodiments, motor 620 (e.g. a servo motor) is mounted to base 602 and is connected to movable plate 610 via drive mechanism 622 coupled to the shaft of the 620. Motor 620 may be driven by controller 900 (see FIGS. 11-14), which may be disposed in the housing 110. As motor 620 rotates, drive mechanism 622 and plate 610 rotate. The motion, in this case rotation, imparted to plate 610 and ultimately pouch 300 may expedite reconstitution of the dry contrast media and improve homogeneity of the reconstituted media. In some embodiments, motor 620 may be driven such that plate 610 rotates in a range of about 1,000 rpm to about 3,000 rpm. In some embodiments, the agitator 600 may be activated for approximately two minutes to fully reconstitute a pouch 300 initially containing 62.3 grams of dry contrast media.

In some non-limiting embodiments, agitator 600 may include at least one heating element configured to apply heat to container 300, the solute contrast media contained within the container, and the diluent during reconstitution. For example, an electric heating mechanism can heat plate 610 to help provide improved mixing (e.g. by helping to speed up the reconstitution process and/or improve dry contrast media dissolving into the diluent/WFI to reconstitute the contrast media). In some non-limiting embodiments, drive mechanism 622 may also be a heating element The heating can be provided via conduction to heat the plate 610 to a pre-selected reconstitution temperature for warming the contents of the container 300 via conduction of heat between the heated plate 610 and the material within the container while agitation occurs for reconstitution.

With reference to FIGS. 33A-33D, plate 610 may include an attachment mechanism for automatically connecting spike 320 of contrast tube 206 to pouch 300 in the manner shown in FIGS. 29A-30C. The attachment mechanism may include rail 640 mounted to plate 610, and carriage 650 slidable on rail 640. Carriage 650 may include clip 652 or other retention member which is secured to spike 320. In preparation for a reconstitution procedure, carriage 650 may be advanced on rail 640 toward pouch 300 until spike 320 is fully seated in internal valve 310 of pouch 300, as shown in FIG. 33D. Carriage 650 may be controlled automatically by controller 300. Agitator 600 may be provided in an enclosure, such as covered by lid 114 within housing 110 as shown in FIGS. 13A-13B, to protect agitator 600 and pouch 300 during reconstitution and/or injection procedures.

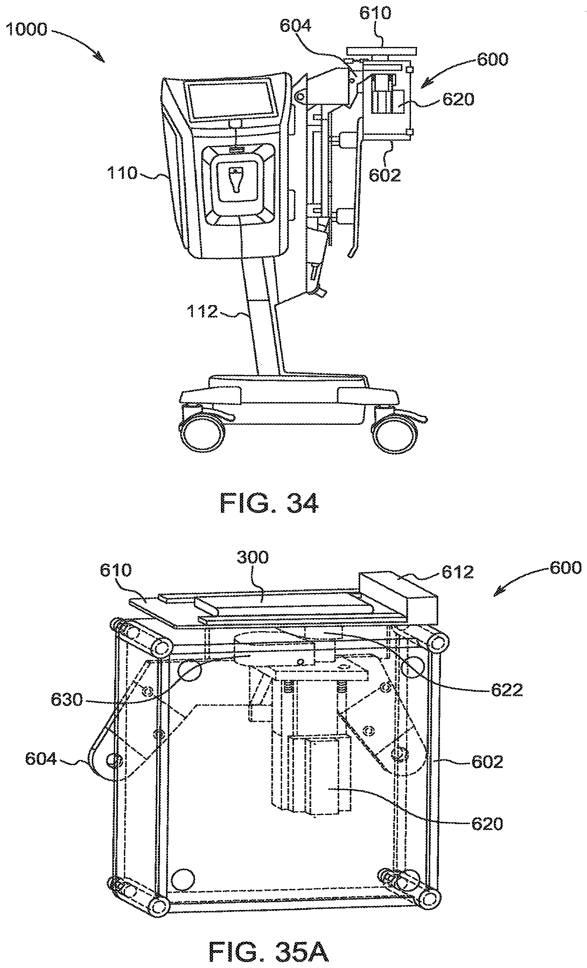

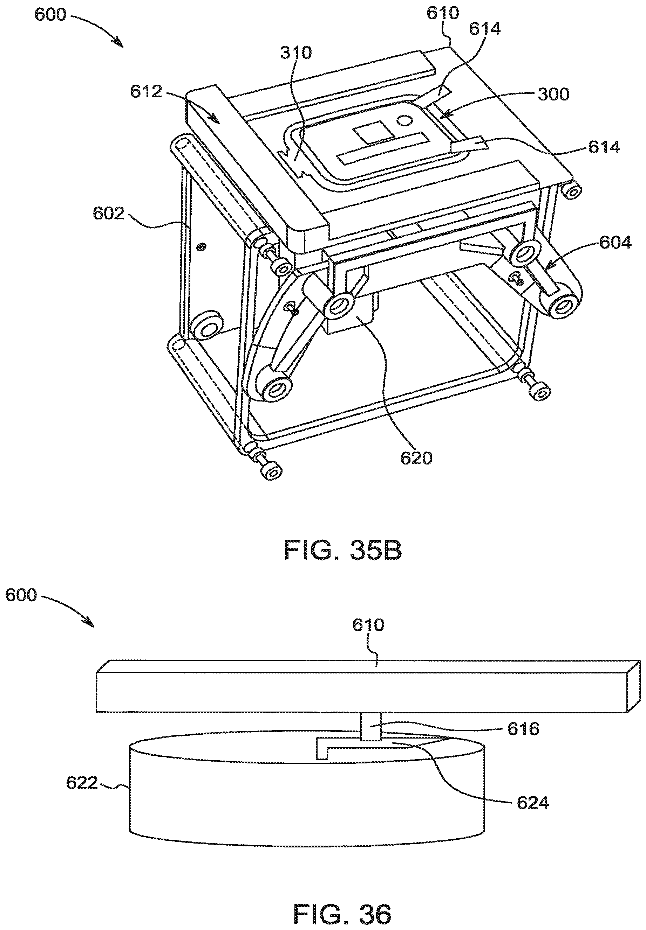

FIGS. 34-37B show alternative embodiments of agitator 600. In these embodiments, components of the agitator are substantially similar to the embodiment of FIGS. 33A-33D, with like numerals used to indicate like components. As shown in FIGS. 34-35B, plate 610 may include one or more clip 614 or other connection features to secure pouch 300 to plate 610. In some embodiments, as shown in FIGS. 35A-37B, plate 610 may include weight 612 to balance the weight of the pouch 300, thereby reducing vibration transmitted to base 602 and ultimately housing 110 and stand 112 as plate 610 is moved. In some embodiments, drive member 622 may also impart lateral movement to plate 610. An example is shown in FIG. 36, in which the drive mechanism includes channel 624 in which retention pin 616 of the plate may slide. As the drive mechanism 622 rotates, retention pin 616 slides along the channel 624. Channel 624 may be of a particular profile, such as a dogleg, to impart a predetermined lateral motion to plate 610. In some embodiments, channel 624 may be configured to allow for lateral movement of about 5 mm to about 10 mm, in certain embodiments about 9 mm. It should be appreciated that in other embodiments, channel 624 could be provided in the underside of plate 610 and retention pin 616 could extend upward from drive mechanism 622 to achieve substantially the same lateral movement profile of the plate 610.

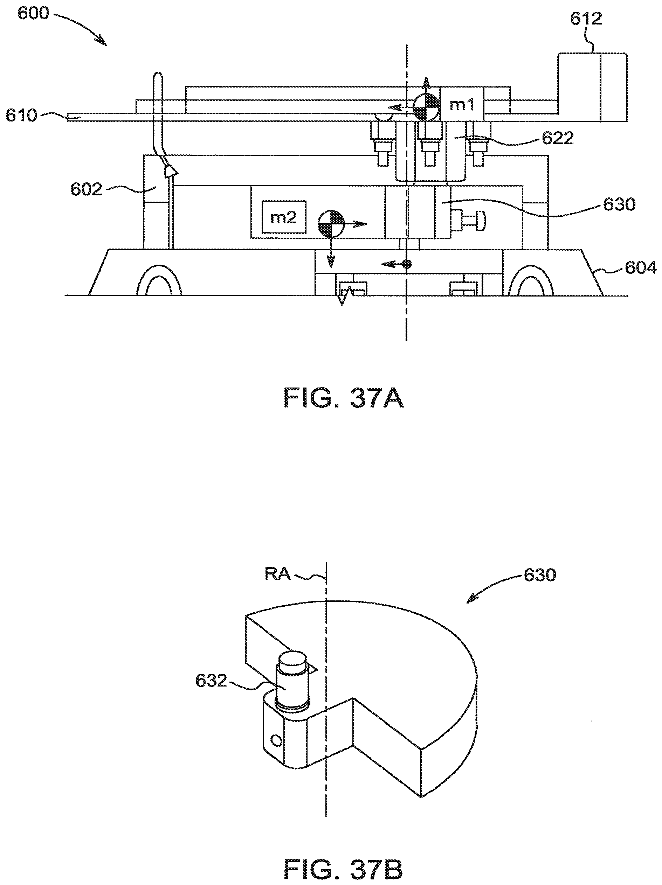

Referring now to FIGS. 37A and 37B, in some embodiments, agitator 600 may include rotating counterweight 630 configured to counteract vibration due to movement of plate 610. Counterweight 630 may be coupled to the shaft of motor 620, and may rotate about rotation axis RA coaxial with an axis of rotation of motor 620. Rotating counterweight 630 may include pin 632 offset from rotational axis RA. Pin 622 may be connected to drive mechanism 622, causing drive mechanism 622 to rotate eccentrically relative to motor 620. This eccentric rotation may consequently be imparted to movable plate 610, and may result in lateral motion of the plate 610.

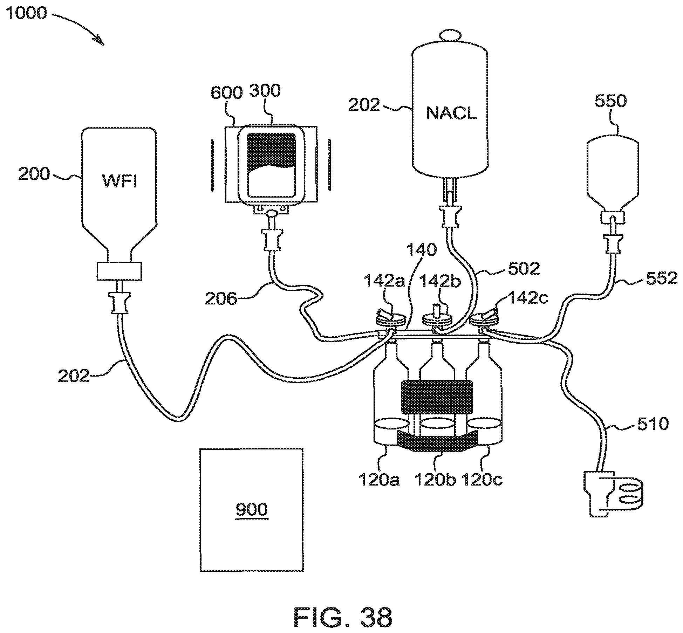

Referring now to FIGS. 38-46, FIG. 38 shows a schematic of a non-limiting embodiment of fluid injector system 1000 in accordance with the preset disclosure in a three barrel configuration. In the illustrated embodiment, components of system 1000 are substantially similar to the embodiment of FIGS. 1-9, with like numerals used to indicate like components. As shown in the illustrated embodiment, system 1000 may include third syringe 120c and corresponding third valve 142c such that the system includes three syringes 120a, 120b, 120c fluidly connected to distribution manifold 140. In this exemplary three barrel embodiment, third valve 142c may be configured to provide fluid communication between one or more of third syringe 120c, liquid contrast media vessel 550, and administration line 510. Accordingly, the flow control assembly may include distribution manifold 140 which may be fluidly connected to bypass tube 552 and liquid contrast media vessel 550 via third valve 120c.

In some embodiments, liquid contrast media vessel may contain contrast media already in liquid form, and may be used during an injection procedure supplemental to or in place of the contrast media reconstituted in pouch 300. The liquid contrast media may also be injectable reconstituted contrast media solution previously produced by system 1000. Use of auxiliary liquid contrast media may be necessary, for example, due to a patient allergy or unavailability of a pouch 300 containing solute contrast media. In other embodiments, liquid contrast media vessel 550 may be used for recycling or reclamation processes associated with reconstituted contrast media solution. In other embodiments, liquid contrast media vessel 550 and bypass tube 552 may be omitted, as shown in FIGS. 1-9.

In the embodiment shown in FIGS. 38-46, where pouch 300 is provided and reconstitution of the solute contrast media in pouch 300 is indicated, system 1000 operates as shown in FIGS. 1-9, with third valve 142c closed as to inhibit flow to and from liquid contrast media vessel 550 and third syringe 120c, unless or until its use is desired for recycling/reclamation. Where an unused portion of the injectable reconstituted contrast media solution from pouch 300 remains in first syringe 120a, controller 900 may be programmed or configured to deliver the unused portion of injectable reconstituted contrast media solution from first syringe 120a into third syringe 120c, and from third syringe 120c to liquid contrast media vessel 550.

First valve 142a may facilitate selective fluid flow control between or more of WFI vessel(s) 200, pouch 300, first syringe 120a, and administration line 510. Second valve 142b may facilitate selective fluid flow control between one or more of second syringe 120b, flushing agent vessel(s) 500, and administration line 510. Third valve 142c may be configured to provide selective fluid communication between one or more of third syringe 120c, liquid contrast media vessel 550, and administration line 510.

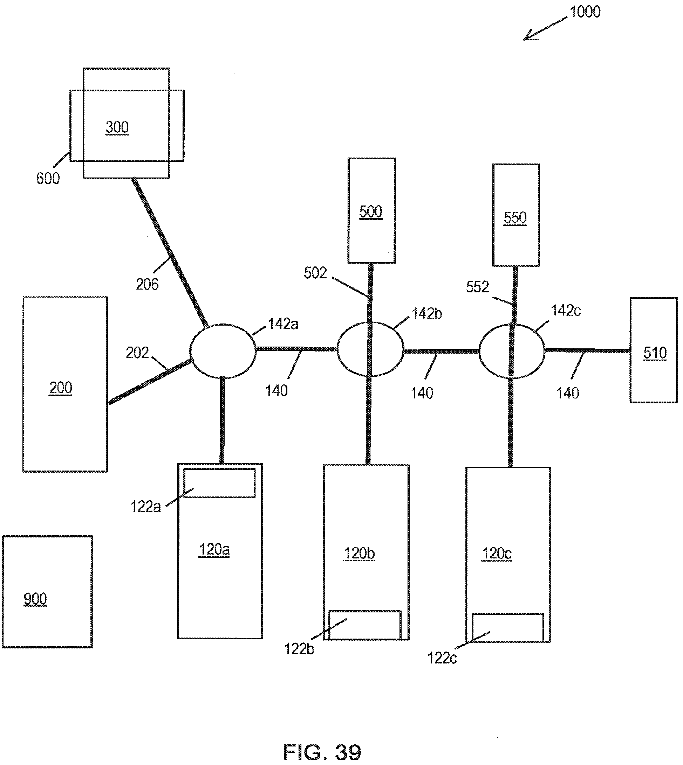

Controller 900 may be configured to actuate valves 142a, 142b, 142c, pistons 122a, 122b, 122c associated with syringes 120a, 120b, 120c; in a prescribed sequence as shown in FIGS. 39-42 in order to deliver liquid contrast media from liquid contrast media vessel 550 to administration line 510, and ultimately, to the patient.

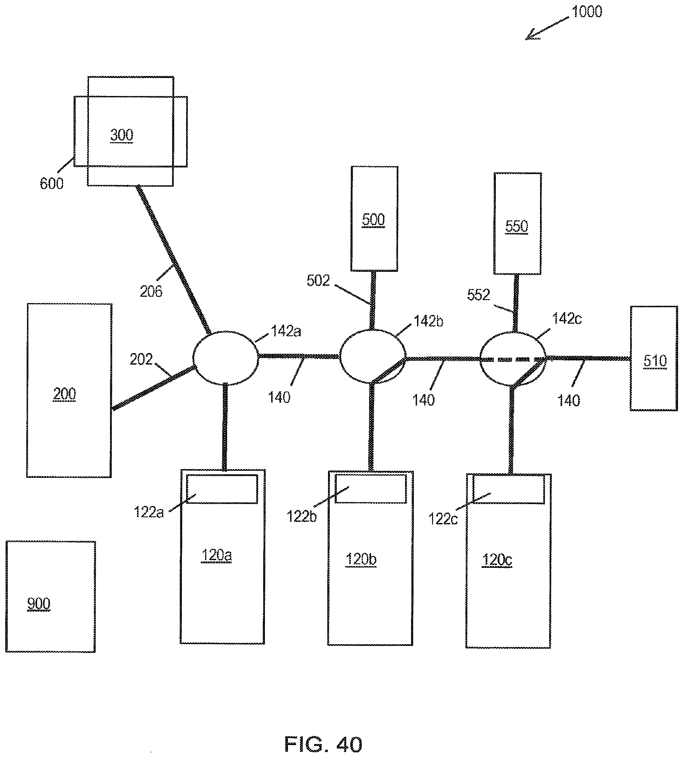

Referring now to FIG. 39, the liquid contrast media from liquid contrast media vessel 550 must first be injected into pouch 300. To that end, third valve 142c may be positioned to establish fluid communication between third syringe 120c and bypass tube 552. Third piston 122c associated with third syringe 120c may be retracted proximally to draw liquid contrast media from liquid contrast media vessel 550 into third syringe 120c, as shown in FIG. 39. Simultaneously, second valve 142b may be positioned to establish fluid communication between second syringe 120b and flushing agent tube 502. Second piston 122b associated with second syringe 120b may be retracted to proximally draw the flushing agent into second syringe 120b, as shown in FIG. 39. As pouch 300 is not being utilized in the illustrative example, first valve 142a is closed and first piston 122a is idle. As shown in FIG. 40, second piston 122b and third piston 122c may be advanced distally to deliver the flushing agent (e.g. saline) from second syringe 120b and liquid contrast media from third syringe 120c to administration line 510 and the patient.

It is contemplated that a filter such as filtration module 400 and or a pump such as pump 450 may be included in bypass tube 502 to filter the liquid contrast media before it enters third syringe 120c. The application of filtration module 400 and/or pump 450 would be the same or similar to that applied to contrast tube 206 as shown and described with reference to FIG. 15. It is also considered that the liquid contrast media may not contain particulates and/or impurities requiring filtration by system 1000, as the liquid contrast media may have already been filtered and/or undergone depyrogenation prior to reconstitution previously executed by system 1000.

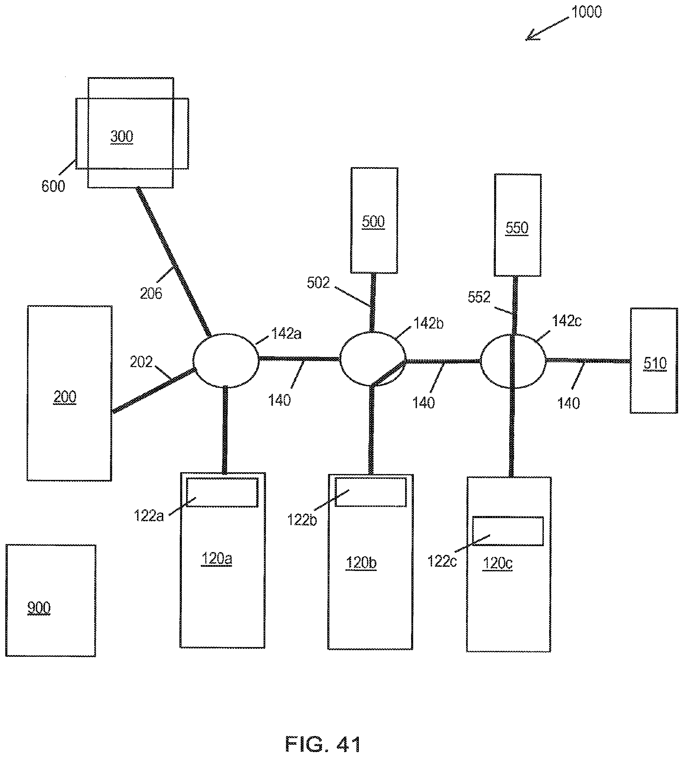

Referring to FIG. 41, third syringe 120c is partially filled with unused contrast media following an injection procedure. As such, third piston 122c is at an intermediate position within third syringe 120c, with the volume between piston 122c and the distal end of syringe 120c filled with unused contrast media. As shown in FIG. 41, third valve 142c is positioned to establish fluid communication between third syringe 120c and liquid contrast media vessel 550.

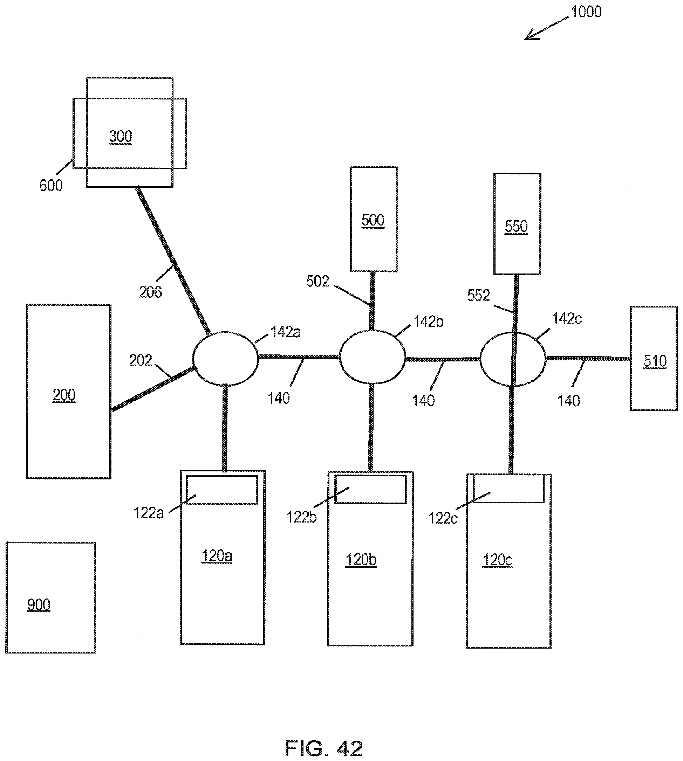

Referring now to FIG. 42, third piston 122c is advanced to deliver the unused liquid contrast media to liquid contrast media vessel 550 for recycling and/or reclamation procedures.

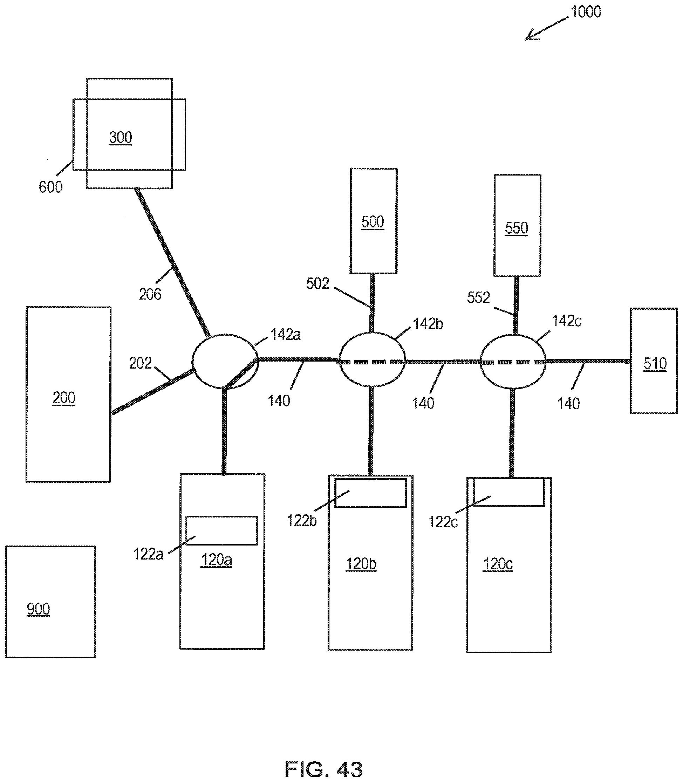

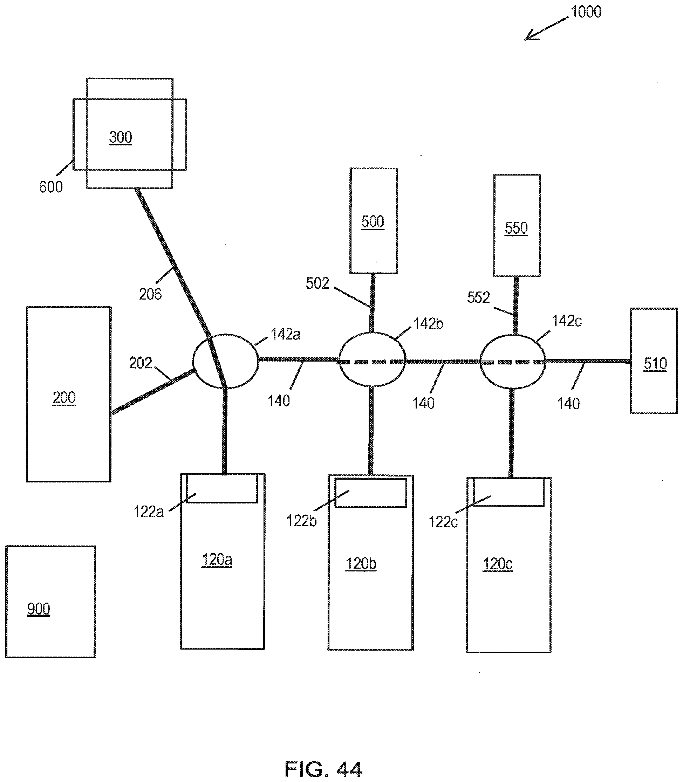

In three barrel embodiments where solute contrast media from pouch 300 is reconstituted and reconstituted contrast media solution is delivered to a patient, first syringe 120a may be partially filled with unused injectable reconstituted contrast media solution, as shown in FIG. 43. As such, first piston 122a is at an intermediate position within first syringe 120a with the volume between piston 122a and the distal end of syringe 120a filled with unused contrast media. As shown in FIG. 44, the first valve 142a is positioned to established fluid communication between third syringe 120a and pouch 300. First piston 122a is then advanced to inject the unused contrast media from first syringe 120a into pouch 300 for recycling and/or reclamation.

A method for reconstituting and injecting a contrast media in a fluid injector system of the illustrated embodiment may include fluidly connecting diluent vessel 200 containing diluent to first syringe 120a, fluidly connecting flushing agent vessel 500 containing a flushing agent to second syringe 120b, fluidly connecting container 300 containing a solute contrast media to first syringe 120a, and fluidly connecting liquid contrast media vessel 550 containing liquid contrast media to third syringe 120c. The method may further include delivering the flushing agent from flushing agent vessel 500 into second syringe 120b, delivering the flushing agent from second syringe 120b into an administration line, delivering the liquid contrast media from liquid contrast media vessel 550 into third syringe 120c, and delivering the liquid contrast media from third syringe 120c into the administration line.

Referring now to FIG. 25, first valve 142a is positioned to establish fluid communication between first syringe 120a and pouch 300. First piston 122a can then be advanced to inject the unused contrast media from first syringe 120a into pouch 300. Pouch 300 may then be disconnected from agitator 600 and from contrast line 206, as shown in FIGS. 29C and 30C. Pouch 300, now filled with unused contrast media, may be sent and/or transported to an appropriate facility for recycling and/or reclaiming. In particular, the iodine and/or other active ingredients may be reclaimed from the unused contrast media. This eliminates the need for hazardous disposal of the unused contrast media.

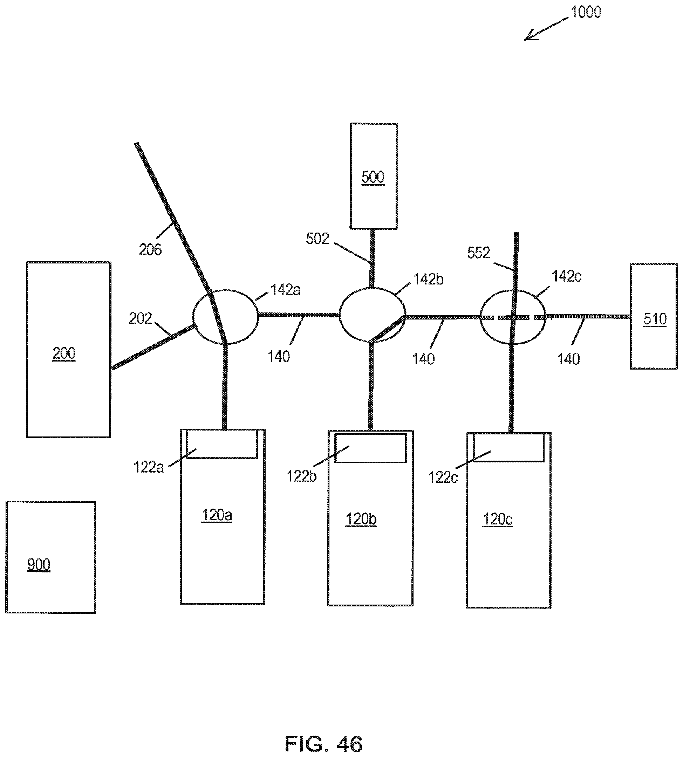

Note that prior to connecting pouch 300 to contrast tube 206, and/or before attaching liquid contrast media vessel 550 to bypass tube 552, the system 1000 is purged of air and primed so that air is not inadvertently injected into the patient. Referring now to FIGS. 45 and 46, an air purge operation of the system 1000 is initiated by retracting each of pistons 122a, 122b, 122c as shown in FIG. 45. First valve 142a is positioned to establish fluid communication between first syringe 120a and the contrast tube 206. Second valve 142b is positioned to establish fluid communication between second syringe 120b and infusion set 510. Third valve 142c is positioned to establish fluid communication between third syringe 120c and the bypass tube 552. Referring now to FIG. 46, pistons 122a, 122b, 122c may be advanced to expel air from syringes 120a, 120b, 120c. In some embodiments, particularly where syringes 120a-120c are part of a "day set", the air purge procedure is performed only once per day after the "day set" is initially connected to the system 1000. Subsequent injections from the same "day set" do not require air purging. Note that pouch 300 is not connected to the contrast tube 206 and liquid contrast media vessel 550 is not connected to bypass tube 552 during the air purging operation, as the purged air must be able to escape the system 1000 via the contrast tube 206, bypass tube 552, and/or infusion set 510. Similar air purge operations are executed by other embodiments of system 1000 described herein, in which the components included in the system dictate the positioning of valves 142a, 142b, etc. to allow fluid connection of syringes 120a, 120b, etc. to the environment outside of system 1000 such that air may be purged from system 1000.

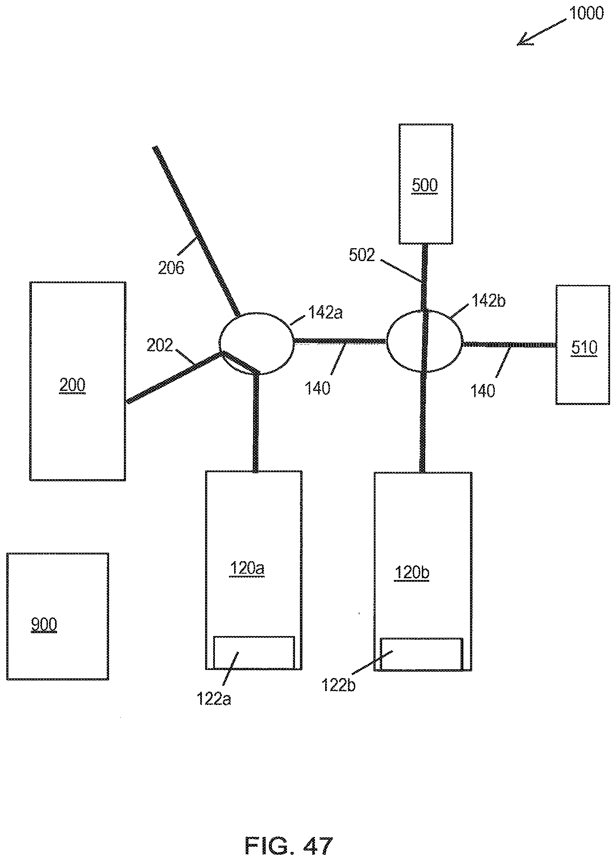

Referring now to FIGS. 47-48, FIGS. 47-48 show system 1000, as shown and described with reference to FIGS. 1-9, executing a priming operation. As shown in FIG. 47, a priming operation may begin by positioning the valves 142a, 142a to draw a diluent such as WFI from diluent vessel 200 and/or a flushing agent from flushing agent vessel 500 into syringes 120a, 120b. As shown in FIG. 47, first valve 142a is positioned to establish fluid communication between first syringe 120a and WFI vessel(s) 200. Second valve 142b is positioned to establish fluid communication between second syringe 120b and flushing agent vessel(s) 500. As shown in FIG. 47, first piston 122a is retracted to draw fluid from the WFI vessel(s) 200 into the first syringe 120a, and second piston 122b is retracted to drawing fluid from the flushing agent vessel(s) 500 into second syringe 120c.

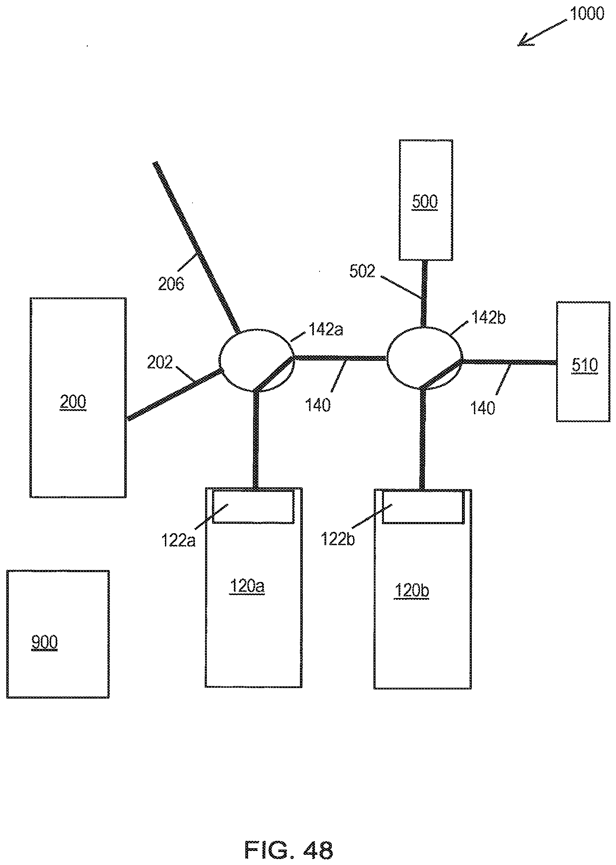

Referring now to FIG. 48, first valve 142a is then positioned to establish fluid connection between first syringe 120a, now loaded with fluid from the WFI vessel(s) 200, and contrast tube 206. Piston 122a is then advanced to inject some of the fluid from the first syringe 120a through contrast tube 206, thereby priming contrast tube 206 with WFI fluid. At this point, contrast tube 206 is suitable for connection to pouch 300. Infusion set 510 is then primed with flushing agent drawn from the flushing agent vessel(s) 500. The second valve 142b is positioned to establish fluid communication between second syringe 120b, which was previously loaded with flushing agent, and infusion set 510. Piston 122c is advanced to inject the flushing agent from second syringe 120b through infusion set 510. Infusion set 510 is now primed and suitable for connection to a vein of the patient. Similar air purge operations are executed by other embodiments of system 1000 described herein, in which the components included in the system dictate the positioning of valves 142a, 142b, etc. to allow fluid connection of syringes 120a, 120b, etc. to prime the various components of system 1000 with the diluent and/or the flushing agent.

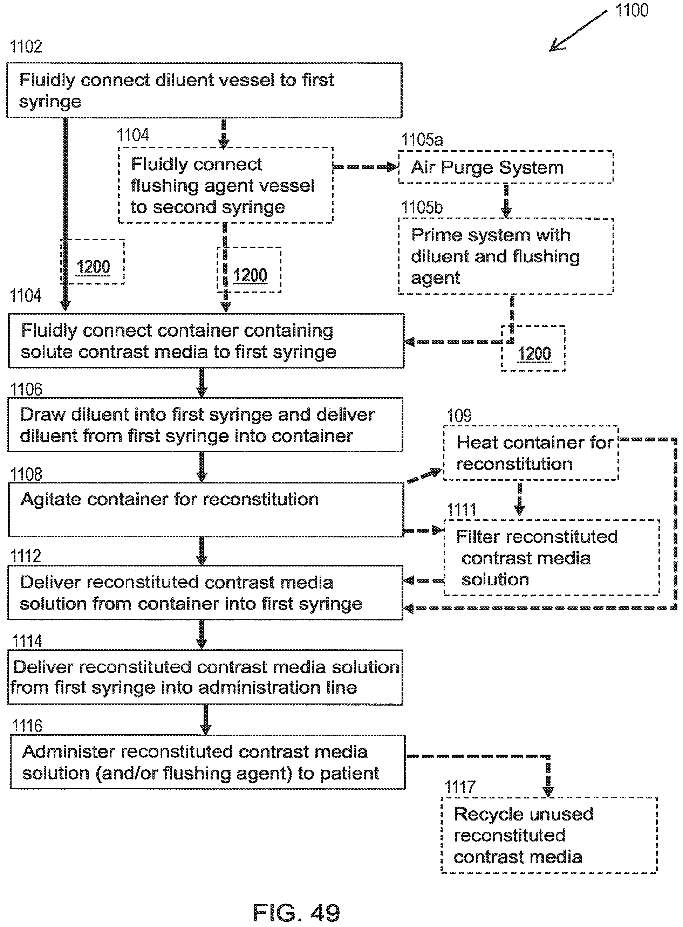

Referring now to FIG. 49, FIG. 49 illustrates a sequence diagram for a method 1100 of reconstituting a solute contrast media and injecting a reconstituted contrast media solution in fluid injector system 1000. Method 1100 may include fluidly connecting diluent vessel 200 containing a diluent such as WFI to first syringe 120a, as shown at step 1102.

In some non-limiting embodiments, such as the embodiment shown in FIGS. 1-9, method 1100 may include fluidly connection flushing agent vessel 500 to second syringe 120b at step 1103. Where step 1103 is included in method 1100, the method may further include delivering the flushing agent from flushing agent vessel 500 into second syringe 120b, and delivering the flushing agent from second syringe 120b into administration line 510 of fluid injector system 1000.

Further, the method may optionally include purging system 1000 of air at step 1105a prior to fluidly connecting container 300 to first syringe 120a as discussed with reference to FIGS. 45-46. The method may also include priming the system at step 1105b with diluent from diluent vessel 200 and flushing agent from flushing agent vessel 500, as discussed with reference to FIGS. 47-48. Step 1105b may occur after fluidly connecting flushing agent vessel 500 to second syringe 120b, but prior to connecting container 300 containing the solute contrast media to first syringe 120a of fluid injector system 1000. Method 1100 may also include method 1200, as shown and described below with reference to FIG. 50, for approving components for use with system 1000.

At step 1104, method 1100 may further include fluidly connecting container 300 containing solute contrast media to first syringe 120a. Then, at step 1106 diluent may be drawn from diluent container 200 into first syringe 120a and delivered from first syringe 120a to container 300 for reconstitution of the solute contrast media. Container 300 may then be agitated at step 1108 to reconstitute the solid contrast media into a reconstituted contrast media solution.

In some non-limiting embodiments, container 300 may also be heated for reconstitution, as shown at step 1109.

In some non-limiting embodiments, after reconstitution, the reconstituted contrast media solution may optionally be filtered, as shown at step 1111. At step 1111, method 1100 may include cleaning the reconstituted contrast media solution by passing the reconstituted contrast media solution through a filter, such as filtration module 400, comprising at least one filter media, before delivering the reconstituted contrast media solution into administration line 510 of fluid injector system 1000.

With continued reference to FIG. 49, the reconstituted contrast media solution may then be delivered from container 300 into first syringe 120a, as shown at step 1112. Then, system 1200 may include delivering the injectable reconstituted contrast media solution from first syringe 120a into administration line 510 at step 1114. At step 1116, the reconstituted contrast media solution, and/or the flushing agent, may be administered to the patient.

In some non-limiting embodiments, after injection, unused reconstituted contrast media may be recycled at optional step 1117 by delivering the unused portion of reconstituted contrast media solution from first syringe 120a into container 300. Alternatively, unused reconstituted contrast media may be recycled at optional step 1117 by delivering an unused portion of the reconstituted contrast media solution from first syringe 120a into liquid contrast media vessel 550.

In some non-limiting embodiments, a method for reconstituting a solute contrast media and injecting a reconstituted contrast media solution in fluid injector system 1000 may further include disconnecting container 300 or liquid contrast media vessel 550 containing the unused portion of the injectable reconstituted contrast media solution from the fluid injector system, and sending the container 300 or liquid contrast media vessel 550 to a recycling facility to reclaim an active ingredient of the reconstituted contrast media solution. Additionally or alternatively, liquid contrast media vessel 550 non containing unused reconstituted contrast media solution may be stored for use in a later injection procedure.

In some non-limiting embodiments, a method for reconstituting a solute contrast media and injecting a reconstituted contrast media solution in fluid injector system 1000 may further include sterilizing the contrast media by heating the solute contrast media in container 300 to a temperature of at least 150 degrees Celsius before fluidly connecting container 300 to first syringe 120a.

As previously described in connection with FIGS. 14 and 27A, pouch 300 may include at least one indicia, such as readable flushing agent identifier 312, that controller 900 may use to validate an injection procedure. WFI vessel(s) 200 and flushing agent vessel(s) 500 may also include similar indicia. Accordingly, flushing agent vessel 500 may include at least one readable flushing agent identifier, and diluent vessel 200 may include at least one readable diluent identifier. Further, system 1000 may include at least one reader or sensor, and the at least one reader may be configured to scan and read the at least one readable solute contrast media identifier, the at least one readable flushing agent identifier, and the at least one readable diluent identifier and communicate identifier information with controller 900.

The reader may be part of, or in communication with, the controller 900 as an operator prepares the system 1000 for use. Controller 900 may be further programmed or configured to process the identifier information and to start and stop fluid communication between one or more of diluent vessel 200, container 300, first syringe 120a, and administration line 510, and between one or more of flushing agent vessel 500, second syringe 120b, and administration line 520 based on the identifier information.

FIG. 50 illustrates a sequence diagram for a method 1200 of validating connection of the pouch 300, WFI vessel(s) 200, and flushing agent vessel(s) 500. At step 1202 of method 1200, the readable diluent identifier of WFI vessel 200 is scanned by the reader. From the indicia, the controller 900 ascertains that the WFI vessel(s) 200 are to be coupled to system 1000, and the controller 900 may further ascertain information about WFI vessel(s) 200 such as type and quantity of liquid contained in WFI vessel(s) 200.

With continued reference to FIG. 50, at step 1204 of method 1200, WFI vessel(s) 200 are attached to housing 110 and/or stand 112 (shown in FIGS. 13A, 13B, and 34) by the operator. In some embodiments, housing 110 and or stand 112 may include an arm from which to hang WFI vessel(s) 200. In some embodiments, the arm may automatically extend from housing 110 and/or stand 112 upon scanning the diluent identifier at step 1202. The operator may then connect the water tube 202 to the WFI port 152 of distribution manifold 140 (as shown in FIGS. 31A and 31B) to establish fluid communication between WFI vessel(s) 200 and first valve 142a.

With continued reference to FIG. 50, at step 1206 of method 1200, the readable flushing agent identifier of flushing agent vessel 500 is scanned by the reader. From the readable flushing agent identifier, controller 900 ascertains that flushing agent vessel(s) 500 are to be coupled to system 1000, and controller 900 may further ascertain information about flushing agent vessel(s) 500 such as type and quantity of liquid contained in flushing agent vessel(s) 500.

With continued reference to FIG. 50, at step 1208 of method 1200, flushing agent vessel(s) 500 are attached to housing 110 and/or stand 112 (shown in FIG. 11) by the operator. In some embodiments, housing 110 and or stand 112 include an arm from which to hang flushing agent vessel(s) 500. In some embodiments, the arm may automatically extend from housing 110 and/or stand 112 upon scanning the readable flushing agent identifier at step 1206. The operator may then connect flushing agent tube 502 to flushing agent port 158 of distribution manifold 140 (as shown in FIGS. 31A and 31B) to establish fluid communication between flushing agent vessel(s) 500 and third valve 142c.

With continued reference to FIG. 50, at step 1210 of the method 1200, the readable contrast media identifier of pouch 300 is scanned by the reader. From the readable contrast media identifier 312 of pouch 300, controller 900 ascertains that pouch 300 is to be coupled to system 1000, and controller 300 may further ascertain information about pouch 300 such as the type and quantity of dry contrast media contained therein, expiration date of pouch 300, and any prior uses of pouch 300.

With continued reference to FIG. 50, at step 1212 of method 1200, controller 900 determines, based on the information gathered from the indicia, such as readable contrast media identifier 312 at step 1210, whether pouch 300 is approved for use based on at least one factor. Factors that controller 900 may use to determine that the pouch is not approved may include, for example, that pouch 300 has reached its expiration date; that pouch 300 contains a contrast media not suitable for a subject injection procedure; that pouch 300 contains an unapproved substance; that pouch 300 does not contain sufficient contrast media to compete the subject injection procedure; that pouch 300 has previously been used; and other factors indicative that the contrast media in pouch 300 cannot safely be delivered to the patient.

If the controller 300 determines at step 1212 that pouch 300 is not approved, controller 900 may abort the injection procedure at step 1214 of the method. The controller 900 may emit a signal (e.g. an audio or visual message) indicating that the pouch 300 is not approved. Controller 900 may prevent performance of an injection procedure until a different, approved pouch 300 is scanned and connected to system 1000.

If controller 900 determines at step 1212 that pouch 300 is approved, pouch 300 may be connected to system 1000 at step 1216 of the method 1200. The operator may position and secure pouch 300 to agitator 600, for example using the clips 614 (see FIGS. 33A, 33B, and 35B). Spike 320 of contrast tube 206 may be inserted into valve 310 of the pouch 300 as shown in FIGS. 29A and 30C, either manually by the operator or automatically by the controller 900 if agitator 600 includes rail 640 and carriage 650 of FIGS. 33A-33D.

With continued reference to FIG. 50, at step 1218 of method 1200, controller 900 reconstitutes the contrast media in pouch 300 by operating valves 142a, 142b, etc. and pistons 122a, 122b, etc. in the sequence shown in FIGS. 2-7, 18-22, and 39-40. With the contrast media reconstituted and loaded into the appropriate syringe (120a, 120b, depending on the embodiment of system 1000), an injection procedure may commence to deliver the reconstituted contrast media (and any prescribed flushing agent) to the patient via infusion set 510.

While several examples of the fluid injector system, devices, and methods for reconstituting a solute contrast media and injecting a reconstituted contrast media solution are shown in the accompanying figures and described in detail hereinabove, other aspects will be apparent to, and readily made by, those skilled in the art without departing from the scope and spirit of the disclosure. Accordingly, the foregoing description is intended to be illustrative rather than restrictive. The invention described hereinabove is defined by the appended claims and all changes to the invention that fall within the meaning and range of equivalency of the claims are to be embraced within their scope.

## Claims

1. A flow control assembly for a fluid injector system for reconstituting a solute contrast media and injecting a reconstituted contrast media solution resulting therefrom, the flow control assembly comprising:
a first syringe;
a second syringe;
an administration line;
a container containing the solute contrast media and comprised of a flexible membrane, a polymeric material capable of withstanding temperatures of at least 150 degrees Celsius, and an internal valve configured to receive a spike of a contrast line and to passively seal the container when the spike is removed from the internal valve;
a first valve configured to provide selective fluid communication between one or more of a diluent vessel containing a diluent, the container, the first syringe, and the administration line.
a second valve configured to provide selective fluid communication between one or more of a flushing agent vessel containing a flushing agent, the second syringe, and the administration line;
an agitator configured to receive the container and comprised of a drive mechanism configured to agitate the container during reconstitution and at least one heating element configured to apply heat to the container; and
a controller programmed or configured to:
control the first valve to deliver the diluent from the diluent vessel into the container to reconstitute the solute contrast media into the reconstituted contrast media solution, deliver the reconstituted contrast media solution into the administration line configured to be fluidly connectable to a patient, and deliver the reconstituted contrast media solution into the patient; and
control the second valve to deliver the flushing agent from the flushing agent vessel into the administration line, and deliver the flushing agent into the patient,
wherein the container is heat treated such that the solute contrast media reaches a temperature of at least 150 degrees Celsius prior to the agitator receiving the container.

2. A flow control assembly for a fluid injector system for reconstituting a solute contrast media and injecting a reconstituted contrast media solution resulting therefrom, the flow control assembly comprising:
a first syringe;
a second syringe;
a third syringe;
a filter comprising at least one filter media;
an administration line;
a container containing the solute contrast media;
a first valve configured to provide selective fluid communication between one or more of the container, a diluent vessel containing a diluent, the first syringe, and the filter;
a second valve configured to provide selective fluid communication between one or more of the filter, the second syringe, and the administration line,
a third valve configured to provide selective fluid communication between one or more of a flushing agent vessel containing a flushing agent, the third syringe, and the administration line;
an agitator configured to receive the container and comprised of a drive mechanism configured to agitate the container during reconstitution; and
a controller programmed or configured to:
control the first valve to deliver the diluent from the diluent vessel into the container, control the agitator to agitate the container to reconstitute the solute contrast media with the diluent into the reconstituted contrast media solution, and control the first valve to deliver the reconstituted contrast media solution into the filter;
control the second valve to deliver the filtered reconstituted contrast media solution into the second syringe and to deliver the filtered reconstituted contrast media solution from the second syringe into the administration line; and
control the third valve to deliver the flushing agent from the flushing agent vessel into the third syringe and to deliver the flushing agent from the third syringe into the administration line,
wherein the administration line is configured to be fluidly connectable to a patient, and
wherein the controller is further programmed or configured to deliver the filtered reconstituted contrast media solution and the flushing agent from the administration line into the patient.

3. A method for reconstituting a solute contrast media and injecting a reconstituted contrast media solution resulting therefrom using a fluid injector system, the method comprising:
fluidly connecting a diluent vessel containing diluent to a first syringe of the fluid injector system;
fluidly connecting a flushing agent vessel containing a flushing agent to a second syringe of the fluid injector system;
fluidly connecting a container containing the solute contrast media to the first syringe of the fluid injector system;
fluidly connecting a liquid contrast media vessel containing liquid contrast media to a third syringe of the fluid injector system;
delivering the flushing agent from the one or more flushing agent vessels into the second syringe;
delivering the flushing agent from the second syringe into an administration line of the fluid injector system.
delivering the liquid contrast media from the liquid contrast media vessel into the third syringe; and
delivering the liquid contrast media from the third syringe into the administration line of the fluid injector system.

4. A container containing a solute contrast media for reconstitution in a fluid injector system, the fluid injector system for use in controlling operation of first and second syringes and corresponding first and second valves in a manifold therefor, the container comprising:
a flexible membrane defining a cavity therein for containing the solute contrast media, the flexible membrane comprising a polymeric material capable of withstanding temperatures of at least 150 degrees Celsius and of enabling the cavity to expand in size; and
an internal valve in communication with the cavity and configured to receive a spike associated with the fluid injector system such that:
(I) upon insertion of the spike into the internal valve, the internal valve opens and thereby under control of the first valve enables alternately:
(a) a diluent to flow from the first syringe through the first valve and the internal valve into the cavity and mix with the solute contrast media to enable formation of a reconstituted contrast media solution therein; and
(b) the reconstituted contrast media solution to flow from the cavity through the internal valve and the first valve into the first syringe from which the fluid injector system is capable of injecting the reconstituted contrast media solution through the first valve through an administration line into a patient; and
(II) upon removal of the spike from the internal valve, the internal valve closes.
